# EUROPEAN PATENT APPLICATION

(11) **EP 4 365 195 A1**
(43) Date of publication of application: **08.05.2024**
(21) Application number: 22833289.6
(22) Date of filing: 30.06.2022
(51) Int. Cl.: C07K 16/10, G01N 33/531, G01N 33/569

(54) **ANTIBODY AGAINST NUCLEOCAPSID PROTEIN OF SARS-COV-2**

(30) Priority: 30.06.2021 JP 2021109546
(71) Applicant: Tauns Laboratories, Inc., Shizuoka 4102325 (JP)
(72) Inventor: SAITO Kenji, Sunto-gun Shizuoka 411-0903 (JP); SUZUKI Sachiko, Sunto-gun Shizuoka 411-0903 (JP); MIYATANI Kazusa, Sunto-gun Shizuoka 411-0903 (JP)
(74) Representative: Eidelsberg, Olivier Nathan
(86) International application number: PCT/JP2022/026279
(87) International publication number: WO 2023/277143

(57) **Abstract**

The purpose of the present invention is to provide an antibody that enables simple, rapid and sensitive detection of SARS-COV-2, i.e., the causative virus of COVID-19 while suppressing nonspecific reactions, and an immunological detection method and a kit comprising the antibody. By using an antibody that binds to the nucleocapsid protein of SARS-COV-2 or an antibody fragment thereof, in particular, an antibody that recognizes a specific region of the protein or an antibody fragment thereof, provided are a specific and sensitive immunological detection method and kit, in particular, a detection method using a sandwich method such as an immunochromatography method or ELISA method, as well as immunochromatographic test strip containing the antibody and a kit containing the test strip.

## Description

### Technical Field

The present invention relates to an antibody against the nucleocapsid protein of the SARS (severe acute respiratory syndrome) coronavirus 2, and uses thereof, for example, a method for immunologically detecting SARS-CoV-2 using the antibody, an immunochromatographic test strip using the antibody, and a kit comprising the test strip.

### Background Art

Four types of coronaviruses of human origin which are NL63, 229E, OC43 and HKU1 have been identified, and account for 10% to 15% of causes of cold. In addition, coronaviruses that infect animals are known. As coronaviruses that are transmitted from animals to humans, the SARS (severe acute respiratory syndrome) coronavirus spread in China's Guangdong Province in 2002 and MARS (Middle East respiratory syndrome) corona virus reported in the Arabian Peninsula in 2012 are previously known.

In December 2019, pneumonia caused by the new coronavirus occurred in Wuhan, Hubei Province in China. The new coronavirus was found to be the Betacoronavirus originating from the same animal as that for the SARS coronavirus and the MARS coronavirus. In February 2019, the new coronavirus was named SARS-CoV-2 by International Committee on Taxonomy of Viruses, and the disease caused by the virus was named COVID-19 (corona virus disease 2019) by World Health Organization (WHO).

The early symptoms of COVID-19 are similar to those of influenza and cold, and include symptoms such as fever, coughing, pharyngodynia, headache, malaise, and dyspnea. Clinical presentations such as a taste disorder and an olfaction disorder have also been reported as initial symptoms. While many patients recover in a week or so after the onset, elderly persons and patients with a malignancy, a chronic obstructive lung disease, a chronic kidney disease, a liver disease, obesity, hyperlipemia, hypertension or diabetes tend to fall into a serious condition at a higher rate as compared to cases of no underlying health condition. Further, in cases of existence of a heart disease, a chronic lung disease, a cerebrovascular disorder or a chronic kidney disease, there tends to be a high rate of death.

For permanent damage, some patients have been reported to have symptoms of a dysosmia, a dysgeusia, dyspnea, malaise and/or coughing even 60 days after the onset of COVID-19, or suffer from continuation of the symptoms even after 120 days.

Infection with SARS-CoV-2 still continues, but even now, it is difficult to supply an anti-virus drug against SARS-CoV-2. It is extremely important that persons infected with SARS-CoV-2 are early identified by a rapid test in order to suppress worsening due to complication. It is very important that infection with SARS-CoV-2 is accurately detected in order to distinguish COVID-19 from other diseases with cold-like symptoms.

As a method for detecting SARS-CoV-2, a nucleic acid detection method using a real-time reverse transcription PCR method is the most common. However, while the nucleic acid detection method exhibits high detection sensitivity, the method has problems of prolonged test time, necessity of use of dedicated equipment and staff skilled in the operations, and high costs. For this reason, a simple, rapid and sensitive detection method is desired for early identification of infected persons. An antigen detection method using an immunochromatography method may be presented as a detection method that is simpler and more rapid than the nucleic acid detection method.

Non-Patent Literature 1 discloses that a nucleocapsid protein of the C-terminal region consisting of the 121st to 419th amino acids of the nucleocapsid protein of SARS-CoV-2 was prepared, and applied for immunization of mice to acquire antibodies against the nucleocapsid protein of SARS-CoV-2. The epitopes of two of the three antibodies obtained have been identified to be the 210th to 231st amino acids and the 382nd to 397th amino acids in the nucleocapsid protein of SARS-CoV-2. On the other hand, the epitope of the remaining one antibody has not been identified, but is presumed to recognize the 335th to 348th amino acids because it does not have cross reactivity with the SARS coronavirus. The Non-Patent Literature 1 discloses that an immunochromatographic test strip was prepared using the acquired antibody, evaluation was performed, and the results showed that the reaction proceeded up to 100 pg/mL for rNP, 5.5 × 10⁵ copies/mL for SARS-CoV-2, and 27.1 in terms of a Ct value of PCR.

Non-Patent Literature 2 discloses a nonspecific reaction by a previously marketed product using an immunochromatography method for detecting SARS-CoV-2. A nucleic acid method was carried out on 57 hospitalized patients including 3 patients confirmed to be positive by an antigen detection method, and 12 facility staff members, and the results showed that all the persons were negative. The test results for the three persons confirmed to be positive are reported to be false-positives caused by a nonspecific reaction in the antigen detection test because on and after the following day, those persons were positive in the antigen detection method and negative in the nucleic acid detection method. Accordingly, it is reported that the antigen detection method is useful for screening, but in view of the frequency of occurrence of the false positive in the antigen detection method, it is desirable to carry out the nucleic acid detection method for detection of SARS-CoV-2.

### Conventional Art Document

### Non-Patent Literature

Non-Patent Literature 1: A. Ryo, et al., Highly specific monoclonal antibodies against SARS-CoV-2 nucleocapsid antigen for accurate diagnosis of COVID-19, Cell Reports Medicine, 29 Oct. 2020, p.33
Non-Patent Literature 2: K. Itoh, H. Tsutani, et ai., False positive results in severe acute respiratory coronavirus 2 (SARS-CoV-2) rapid antigen tests for inpatients, Journal of Infection and Chemotherapy, 22 Mar. 2021

### Summary of Invention

### Technical problem

However, with consideration given to a screening test and the like, a more sensitive, more reliable and simpler antigen test is desired.

An object of the present invention is to provide an immunological detection method that enables simple, rapid and sensitive detection of SARS-CoV-2, a causative virus of COVID-19, with an antibody that sufficiently specifically reacts, as well as an immunochromatographic test strip comprising the antibody, and a kit comprising the test strip.

### Solution to Problem

In view of the circumstances described above, the present inventors have extensively conducted studies, and resultantly obtained an antibody against the nucleocapsid protein of SARS-CoV-2, leading to completion of the present invention.

The present invention includes the following aspects.
[1] A method for detecting a presence of SARS-CoV-2 contained in a test sample, which comprises performing an immunoassay using an antibody or a fragment thereof that reacts with a nucleocapsid protein of the SARS-CoV-2.
[2] The method for detecting SARS-CoV-2 according to [1], wherein the immunoassay comprises contacting the test sample with the antibody or the fragment thereof.
[3] The method for detecting SARS-CoV-2 according to [2], wherein the immunoassay is a sandwich method using the two different antibodies or fragments thereof.
[4] The method for detecting SARS-CoV-2 according to [3], wherein the sandwich method is an immunochromatography method or an ELISA method.
[5] The method for detecting SARS-CoV-2 according to any one of [1] to [4], wherein the antibody or fragment thereof is selected from the antibodies or fragments thereof described in (1) to (20) below:
   (1) an antibody in which CDR1, CDR2 and CDR3 of a heavy chain comprise amino acid sequences set forth as SEQ ID NOS: 2, 3 and 4, respectively, and CDR1, CDR2 and CDR3 of a light chain comprise amino acid sequences set forth as SEQ ID NOS: 5, 6 and 7, respectively, or a fragment thereof;
   (2) an antibody in which CDR1, CDR2 and CDR3 of a heavy chain comprise amino acid sequences set forth as SEQ ID NOS: 8, 9 and 10, respectively, and CDR1, CDR2 and CDR3 of a light chain comprise amino acid sequences set forth as SEQ ID NOS: 11, 12 and 13, respectively, or a fragment thereof;
   (3) an antibody in which CDR1, CDR2 and CDR3 of a heavy chain comprise amino acid sequences set forth as SEQ ID NOS: 14, 15 and 16, respectively, and CDR1, CDR2 and CDR3 of a light chain comprise amino acid sequences set forth as SEQ ID NOS: 17, 18 and 19, respectively, or a fragment thereof;
   (4) an antibody in which CDR1, CDR2 and CDR3 of a heavy chain comprise amino acid sequences set forth as SEQ ID NOS: 20, 21 and 22, respectively, and CDR1, CDR2 and CDR3 of a light chain comprise amino acid sequences set forth as SEQ ID NOS: 23, 24 and 25, respectively, or a fragment thereof;
   (5) an antibody in which CDR1, CDR2 and CDR3 of a heavy chain comprise amino acid sequences set forth as SEQ ID NOS: 26, 27 and 28, respectively, and CDR1, CDR2 and CDR3 of a light chain comprise amino acid sequences set forth as SEQ ID NOS: 29, 30 and 31, respectively, or a fragment thereof;
   (6) an antibody in which CDR1, CDR2 and CDR3 of a heavy chain comprise amino acid sequences set forth as SEQ ID NOS: 32, 33 and 34, respectively, and CDR1, CDR2 and CDR3 of a light chain comprise amino acid sequences set forth as SEQ ID NOS: 35, 36 and 37, respectively, or a fragment thereof;
   (7) an antibody in which CDR1, CDR2 and CDR3 of a heavy chain comprise amino acid sequences set forth as SEQ ID NOS: 38, 39 and 40, respectively, and CDR1, CDR2 and CDR3 of a light chain comprise amino acid sequences set forth as SEQ ID NOS: 41, 42 and 43, respectively, or a fragment thereof;
   (8) an antibody in which CDR1, CDR2 and CDR3 of a heavy chain comprise amino acid sequences set forth as SEQ ID NOS: 44, 45 and 46, respectively, and CDR1, CDR2 and CDR3 of a light chain comprise amino acid sequences set forth as SEQ ID NOS: 47, 48 and 49, respectively, or a fragment thereof;
   (9) an antibody in which CDR1, CDR2 and CDR3 of a heavy chain comprise amino acid sequences set forth as SEQ ID NOS: 50, 51 and 52, respectively, and CDR1, CDR2 and CDR3 of a light chain comprise amino acid sequences set forth as SEQ ID NOS: 53, 54 and 55, respectively, or a fragment thereof;
   (10) an antibody in which CDR1, CDR2 and CDR3 of a heavy chain comprise amino acid sequences set forth as SEQ ID NOS: 56, 57 and 58, respectively, and CDR1, CDR2 and CDR3 of a light chain comprise amino acid sequences set forth as SEQ ID NOS: 59, 60 and 61, respectively, or a fragment thereof;
   (11) an antibody in which CDR1, CDR2 and CDR3 of a heavy chain comprise amino acid sequences set forth as SEQ ID NOS: 62, 63 and 64, respectively, and CDR1, CDR2 and CDR3 of a light chain comprise amino acid sequences set forth as SEQ ID NOS: 65, 66 and 67, respectively, or a fragment thereof;
   (12) an antibody in which CDR1, CDR2 and CDR3 of a heavy chain comprise amino acid sequences set forth as SEQ ID NOS: 68, 69 and 70, respectively, and CDR1, CDR2 and CDR3 of a light chain comprise amino acid sequences set forth as SEQ ID NOS: 71, 72 and 73, respectively, or a fragment thereof;
   (13) an antibody in which CDR1, CDR2 and CDR3 of a heavy chain comprise amino acid sequences set forth as SEQ ID NOS: 74, 75 and 76, respectively, and CDR1, CDR2 and CDR3 of a light chain comprise amino acid sequences set forth as SEQ ID NOS: 77, 78 and 79, respectively, or a fragment thereof;
   (14) an antibody in which CDR1, CDR2 and CDR3 of a heavy chain comprise amino acid sequences set forth as SEQ ID NOS: 80, 81 and 82, respectively, and CDR1, CDR2 and CDR3 of a light chain comprise amino acid sequences set forth as SEQ ID NOS: 83, 84 and 85, respectively, or a fragment thereof;
   (15) an antibody in which CDR1, CDR2 and CDR3 of a heavy chain comprise amino acid sequences set forth as SEQ ID NOS: 86, 87 and 88, respectively, and CDR1, CDR2 and CDR3 of a light chain comprise amino acid sequences set forth as SEQ ID NOS: 89, 90 and 91, respectively, or a fragment thereof;
   (16) an antibody in which CDR1, CDR2 and CDR3 of a heavy chain comprise amino acid sequences set forth as SEQ ID NOS: 92, 93 and 94, respectively, and CDR1, CDR2 and CDR3 of a light chain comprise amino acid sequences set forth as SEQ ID NOS: 95, 96 and 97, respectively, or a fragment thereof;
   (17) an antibody in which CDR1, CDR2 and CDR3 of a heavy chain comprise amino acid sequences set forth as SEQ ID NOS: 98, 99 and 100, respectively, and CDR1, CDR2 and CDR3 of a light chain comprise amino acid sequences set forth as SEQ ID NOS: 101, 102 and 103, respectively, or a fragment thereof;
   (18) an antibody in which CDR1, CDR2 and CDR3 of a heavy chain comprise amino acid sequences set forth as SEQ ID NOS: 104, 105 and 106, respectively, and CDR1, CDR2 and CDR3 of a light chain comprise amino acid sequences set forth as SEQ ID NOS: 107, 108 and 109, respectively, or a fragment thereof;
   (19) an antibody in which CDR1, CDR2 and CDR3 of a heavy chain comprise amino acid sequences set forth as SEQ ID NOS: 110, 111 and 112, respectively, and CDR1, CDR2 and CDR3 of a light chain comprise amino acid sequences set forth as SEQ ID NOS: 113, 114 and 115, respectively, or a fragment thereof; and
   (20) an antibody in which CDR1, CDR2 and CDR3 of a heavy chain comprise amino acid sequences set forth as SEQ ID NOS: 116, 117 and 118, respectively, and CDR1, CDR2 and CDR3 of a light chain comprise amino acid sequences set forth as SEQ ID NOS: 119, 120 and 121, respectively, or a fragment thereof.
[6] The method for detecting SARS-CoV-2 according to any one of [1] to [4], wherein the test sample is a biological sample.
[7] An immunochromatographic test strip for detecting SARS-CoV-2, comprising a first antibody or a fragment thereof capable of reacting with a nucleocapsid protein of SARS-CoV-2, a second antibody or a fragment thereof that reacts with the nucleocapsid protein, and a membrane carrier, wherein the first antibody or fragment thereof is previously fixed at a predetermined position on the membrane carrier to form a capture portion, and the second antibody or fragment thereof is labeled with a labeling substance and provided at a distance from the capture portion so as to be chromatographically developable in the membrane carrier.
[8] The immunochromatographic test strip according to [7], wherein at least one of the first antibody or fragment thereof and the second antibody or fragment thereof is a monoclonal antibody or fragment thereof.
[9] The immunochromatographic test strip according to [7] or [8], wherein the monoclonal antibody or fragment thereof is selected from the antibodies or fragments thereof described in (1) to (20) below:
   (1) an antibody in which CDR1, CDR2 and CDR3 of a heavy chain comprise amino acid sequences set forth as SEQ ID NOS: 2, 3 and 4, respectively, and CDR1, CDR2 and CDR3 of a light chain comprise amino acid sequences set forth as SEQ ID NOS: 5, 6 and 7, respectively, or a fragment thereof;
   (2) an antibody in which CDR1, CDR2 and CDR3 of a heavy chain comprise amino acid sequences set forth as SEQ ID NOS: 8, 9 and 10, respectively, and CDR1, CDR2 and CDR3 of a light chain comprise amino acid sequences set forth as SEQ ID NOS: 11, 12 and 13, respectively, or a fragment thereof;
   (3) an antibody in which CDR1, CDR2 and CDR3 of a heavy chain comprise amino acid sequences set forth as SEQ ID NOS: 14, 15 and 16, respectively, and CDR1, CDR2 and CDR3 of a light chain comprise amino acid sequences set forth as SEQ ID NOS: 17, 18 and 19, respectively, or a fragment thereof;
   (4) an antibody in which CDR1, CDR2 and CDR3 of a heavy chain comprise amino acid sequences set forth as SEQ ID NOS: 20, 21 and 22, respectively, and CDR1, CDR2 and CDR3 of a light chain comprise amino acid sequences set forth as SEQ ID NOS: 23, 24 and 25, respectively, or a fragment thereof;
   (5) an antibody in which CDR1, CDR2 and CDR3 of a heavy chain comprise amino acid sequences set forth as SEQ ID NOS: 26, 27 and 28, respectively, and CDR1, CDR2 and CDR3 of a light chain comprise amino acid sequences set forth as SEQ ID NOS: 29, 30 and 31, respectively, or a fragment thereof;
   (6) an antibody in which CDR1, CDR2 and CDR3 of a heavy chain comprise amino acid sequences set forth as SEQ ID NOS: 32, 33 and 34, respectively, and CDR1, CDR2 and CDR3 of a light chain comprise amino acid sequences set forth as SEQ ID NOS: 35, 36 and 37, respectively, or a fragment thereof;
   (7) an antibody in which CDR1, CDR2 and CDR3 of a heavy chain comprise amino acid sequences set forth as SEQ ID NOS: 38, 39 and 40, respectively, and CDR1, CDR2 and CDR3 of a light chain comprise amino acid sequences set forth as SEQ ID NOS: 41, 42 and 43, respectively, or a fragment thereof;
   (8) an antibody in which CDR1, CDR2 and CDR3 of a heavy chain comprise amino acid sequences set forth as SEQ ID NOS: 44, 45 and 46, respectively, and CDR1, CDR2 and CDR3 of a light chain comprise amino acid sequences set forth as SEQ ID NOS: 47, 48 and 49, respectively, or a fragment thereof;
   (9) an antibody in which CDR1, CDR2 and CDR3 of a heavy chain comprise amino acid sequences set forth as SEQ ID NOS: 50, 51 and 52, respectively, and CDR1, CDR2 and CDR3 of a light chain comprise amino acid sequences set forth as SEQ ID NOS: 53, 54 and 55, respectively, or a fragment thereof;
   (10) an antibody in which CDR1, CDR2 and CDR3 of a heavy chain comprise amino acid sequences set forth as SEQ ID NOS: 56, 57 and 58, respectively, and CDR1, CDR2 and CDR3 of a light chain comprise amino acid sequences set forth as SEQ ID NOS: 59, 60 and 61, respectively, or a fragment thereof;
   (11) an antibody in which CDR1, CDR2 and CDR3 of a heavy chain comprise amino acid sequences set forth as SEQ ID NOS: 62, 63 and 64, respectively, and CDR1, CDR2 and CDR3 of a light chain comprise amino acid sequences set forth as SEQ ID NOS: 65, 66 and 67, respectively, or a fragment thereof;
   (12) an antibody in which CDR1, CDR2 and CDR3 of a heavy chain comprise amino acid sequences set forth as SEQ ID NOS: 68, 69 and 70, respectively, and CDR1, CDR2 and CDR3 of a light chain comprise amino acid sequences set forth as SEQ ID NOS: 71, 72 and 73, respectively, or a fragment thereof;
   (13) an antibody in which CDR1, CDR2 and CDR3 of a heavy chain comprise amino acid sequences set forth as SEQ ID NOS: 74, 75 and 76, respectively, and CDR1, CDR2 and CDR3 of a light chain comprise amino acid sequences set forth as SEQ ID NOS: 77, 78 and 79, respectively, or a fragment thereof;
   (14) an antibody in which CDR1, CDR2 and CDR3 of a heavy chain comprise amino acid sequences set forth as SEQ ID NOS: 80, 81 and 82, respectively, and CDR1, CDR2 and CDR3 of a light chain comprise amino acid sequences set forth as SEQ ID NOS: 83, 84 and 85, respectively, or a fragment thereof;
   (15) an antibody in which CDR1, CDR2 and CDR3 of a heavy chain comprise amino acid sequences set forth as SEQ ID NOS: 86, 87 and 88, respectively, and CDR1, CDR2 and CDR3 of a light chain comprise amino acid sequences set forth as SEQ ID NOS: 89, 90 and 91, respectively, or a fragment thereof;
   (16) an antibody in which CDR1, CDR2 and CDR3 of a heavy chain comprise amino acid sequences set forth as SEQ ID NOS: 92, 93 and 94, respectively, and CDR1, CDR2 and CDR3 of a light chain comprise amino acid sequences set forth as SEQ ID NOS: 95, 96 and 97, respectively, or a fragment thereof;
   (17) an antibody in which CDR1, CDR2 and CDR3 of a heavy chain comprise amino acid sequences set forth as SEQ ID NOS: 98, 99 and 100, respectively, and CDR1, CDR2 and CDR3 of a light chain comprise amino acid sequences set forth as SEQ ID NOS: 101, 102 and 103, respectively, or a fragment thereof;
   (18) an antibody in which CDR1, CDR2 and CDR3 of a heavy chain comprise amino acid sequences set forth as SEQ ID NOS: 104, 105 and 106, respectively, and CDR1, CDR2 and CDR3 of a light chain comprise amino acid sequences set forth as SEQ ID NOS: 107, 108 and 109, respectively, or a fragment thereof;
   (19) an antibody in which CDR1, CDR2 and CDR3 of a heavy chain comprise amino acid sequences set forth as SEQ ID NOS: 110, 111 and 112, respectively, and CDR1, CDR2 and CDR3 of a light chain comprise amino acid sequences set forth as SEQ ID NOS: 113, 114 and 115, respectively, or a fragment thereof; and
   (20) an antibody in which CDR1, CDR2 and CDR3 of a heavy chain comprise amino acid sequences set forth as SEQ ID NOS: 116, 117 and 118, respectively, and CDR1, CDR2 and CDR3 of a light chain comprise amino acid sequences set forth as SEQ ID NOS: 119, 120 and 121, respectively, or a fragment thereof.
[10] A SARS-CoV-2 detection kit comprising at least the immunochromatographic test strip for detecting SARS-CoV-2 according to [7], and a developing solvent for developing an analyte.
[11] An antibody or fragment thereof for detecting a presence of SARS-CoV-2 contained in a test sample, comprising an antibody or fragment thereof that binds to a nucleocapsid protein of the SARS-CoV-2.
[12] The antibody or fragment thereof according to [11], wherein the antibody is a monoclonal antibody.
[13] The antibody or fragment thereof according to [12], wherein CDR1, CDR2 and CDR3 of a heavy chain comprise amino acid sequences set forth as SEQ ID NOS: 2, 3 and 4, respectively, and CDR1, CDR2 and CDR3 of a light chain comprise amino acid sequences set forth as SEQ ID NOS: 5, 6 and 7, respectively.
[14] The antibody or fragment thereof according to [12], wherein CDR1, CDR2 and CDR3 of a heavy chain comprise amino acid sequences set forth as SEQ ID NOS: 8, 9 and 10, respectively, and CDR1, CDR2 and CDR3 of a light chain comprise amino acid sequences set forth as SEQ ID NOS: 11, 12 and 13, respectively.
[15] The antibody or fragment thereof according to [12], wherein CDR1, CDR2 and CDR3 of a heavy chain comprise amino acid sequences set forth as SEQ ID NOS: 14, 15 and 16, respectively, and CDR1, CDR2 and CDR3 of a light chain comprise amino acid sequences set forth as SEQ ID NOS: 17, 18 and 19, respectively.
[16] The antibody or fragment thereof according to [12], wherein CDR1, CDR2 and CDR3 of a heavy chain comprise amino acid sequences set forth as SEQ ID NOS: 20, 21 and 22, respectively, and CDR1, CDR2 and CDR3 of a light chain comprise amino acid sequences set forth as SEQ ID NOS: 23, 24 and 25, respectively.
[17] The antibody or fragment thereof according to [12], wherein CDR1, CDR2 and CDR3 of a heavy chain comprise amino acid sequences set forth as SEQ ID NOS: 26, 27 and 28, respectively, and CDR1, CDR2 and CDR3 of a light chain comprise amino acid sequences set forth as SEQ ID NOS: 29, 30 and 31, respectively.
[18] The antibody or fragment thereof according to [12], wherein CDR1, CDR2 and CDR3 of a heavy chain comprise amino acid sequences set forth as SEQ ID NOS: 32, 33 and 34, respectively, and CDR1, CDR2 and CDR3 of a light chain comprise amino acid sequences set forth as SEQ ID NOS: 35, 36 and 37, respectively.
[19] The antibody or fragment thereof according to [12], wherein CDR1, CDR2 and CDR3 of a heavy chain comprise amino acid sequences set forth as SEQ ID NOS: 38, 39 and 40, respectively, and CDR1, CDR2 and CDR3 of a light chain comprise amino acid sequences set forth as SEQ ID NOS: 41, 42 and 43, respectively.
[20] The antibody or fragment thereof according to [12], wherein CDR1, CDR2 and CDR3 of a heavy chain comprise amino acid sequences set forth as SEQ ID NOS: 44, 45 and 46, respectively, and CDR1, CDR2 and CDR3 of a light chain comprise amino acid sequences set forth as SEQ ID NOS: 47, 48 and 49, respectively.
[21] The antibody or fragment thereof according to [12], wherein CDR1, CDR2 and CDR3 of a heavy chain comprise amino acid sequences set forth as SEQ ID NOS: 50, 51 and 52, respectively, and CDR1, CDR2 and CDR3 of a light chain comprise amino acid sequences set forth as SEQ ID NOS: 53, 54 and 55, respectively.
[22] The antibody or fragment thereof according to [12], wherein CDR1, CDR2 and CDR3 of a heavy chain comprise amino acid sequences set forth as SEQ ID NOS: 56, 57 and 58, respectively, and CDR1, CDR2 and CDR3 of a light chain comprise amino acid sequences set forth as SEQ ID NOS: 59, 60 and 61, respectively.
[23] The antibody or fragment thereof according to [12], whereinCDR1, CDR2 and CDR3 of a heavy chain comprise amino acid sequences set forth as SEQ ID NOS: 62, 63 and 64, respectively, and CDR1, CDR2 and CDR3 of a light chain comprise amino acid sequences set forth as SEQ ID NOS: 65, 66 and 67, respectively.
[24] The antibody or fragment thereof according to [12], wherein CDR1, CDR2 and CDR3 of a heavy chain comprise amino acid sequences set forth as SEQ ID NOS: 68, 69 and 70, respectively, and CDR1, CDR2 and CDR3 of a light chain comprise amino acid sequences set forth as SEQ ID NOS: 71, 72 and 73, respectively.
[25] The antibody or fragment thereof according to [12], wherein CDR1, CDR2 and CDR3 of a heavy chain comprise amino acid sequences set forth as SEQ ID NOS: 74, 75 and 76, respectively, and CDR1, CDR2 and CDR3 of a light chain comprise amino acid sequences set forth as SEQ ID NOS: 77, 78 and 79, respectively.
[26] The antibody or fragment thereof according to [12], wherein CDR1, CDR2 and CDR3 of a heavy chain comprise amino acid sequences set forth as SEQ ID NOS: 80, 81 and 82, respectively, and CDR1, CDR2 and CDR3 of a light chain comprise amino acid sequences set forth as SEQ ID NOS: 83, 84 and 85, respectively.
[27] The antibody or fragment thereof according to [12], wherein CDR1, CDR2 and CDR3 of a heavy chain comprise amino acid sequences set forth as SEQ ID NOS: 86, 87 and 88, respectively, and CDR1, CDR2 and CDR3 of a light chain comprise amino acid sequences set forth as SEQ ID NOS: 89, 90 and 91, respectively.
[28] The antibody or fragment thereof according to [12], wherein CDR1, CDR2 and CDR3 of a heavy chain comprise amino acid sequences set forth as SEQ ID NOS: 92, 93 and 94, respectively, and CDR1, CDR2 and CDR3 of a light chain comprise amino acid sequences set forth as SEQ ID NOS: 95, 96 and 97, respectively.
[29] The antibody or fragment thereof according to [12], wherein CDR1, CDR2 and CDR3 of a heavy chain comprise amino acid sequences set forth as SEQ ID NOS: 98, 99 and 100, respectively, and CDR1, CDR2 and CDR3 of a light chain comprise amino acid sequences set forth as SEQ ID NOS: 101, 102 and 103, respectively.
[30] The antibody or fragment thereof according to [12], wherein CDR1, CDR2 and CDR3 of a heavy chain comprise amino acid sequences set forth as SEQ ID NOS: 104, 105 and 106, respectively, and CDR1, CDR2 and CDR3 of a light chain comprise amino acid sequences set forth as SEQ ID NOS: 107, 108 and 109, respectively.
[31] The antibody or fragment thereof according to [12], wherein CDR1, CDR2 and CDR3 of a heavy chain comprise amino acid sequences set forth as SEQ ID NOS: 110, 111 and 112, respectively, and CDR1, CDR2 and CDR3 of a light chain comprise amino acid sequences set forth as SEQ ID NOS: 113, 114 and 115, respectively.
[32] The antibody or fragment thereof according to [12], wherein CDR1, CDR2 and CDR3 of a heavy chain comprise amino acid sequences set forth as SEQ ID NOS: 116, 117 and 118, respectively, and CDR1, CDR2 and CDR3 of a light chain comprise amino acid sequences set forth as SEQ ID NOS: 119, 120 and 121, respectively.

### Advantageous Effects of Invention

According to the present invention, it is possible to detect the nucleocapsid protein of SARS-CoV-2 using an antibody against the nucleocapsid protein of SARS-CoV-2. In addition, it is possible to detect SARS-CoV-2 with high sensitivity while suppressing a nonspecific reaction in immunological detection. Further, it is possible to provide an immunochromatographic test strip that enables specific and sensitive detection of SARS-CoV-2, and a kit comprising the test strip.

### Brief Description of Drawings

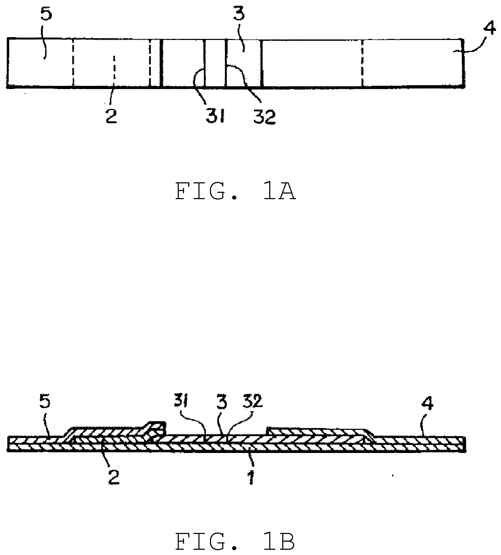

FIG. 1A is a plan view of an immunochromatographic test strip, and FIG. 1B is a longitudinal-direction sectional view of the immunochromatographic test strip shown in FIG. 1A.

### Description of Embodiments

In the present invention, steps in production of an antibody and a detection method using the antibody are each carried out in accordance with an immunological approach known *per se.*

### Antibody

The antibody of the present invention specifically binds to the nucleocapsid protein of SARS-CoV-2, and may be either a polyclonal antibody or a monoclonal antibody.

A polyclonal antibody specific to the nucleocapsid protein of SARS-CoV-2 can be obtained from an antiserum resulting from immunization of an animal with an immunizing antigen which may be, for example, a nucleocapsid protein extracted and purified from SARS-CoV-2, or a nucleocapsid protein that is extracted and purified after being expressed in a genetic engineering manner by introducing a gene of a nucleocapsid protein into Escherichia coli or the like, or a polypeptide forming a part of the nucleocapsid protein.

A monoclonal antibody specific to the nucleocapsid protein of SARS-CoV-2 can be obtained by, for example, the following method. First, as described above, a nucleocapsid protein extracted and purified from SARS-CoV-2, or a nucleocapsid protein that is extracted and purified after expressed in a genetic engineering manner, or a polypeptide forming a part of the nucleocapsid protein is used as an immunizing antigen to immunize an animal such as a mouse, a rat, a rabbit or a chicken. When a mouse is immunized, a hybridoma obtained by fusing a spleen cell of the immunized animal with a myeloma cell is selected in a HAT-containing medium, and then proliferated. The culture supernatant of the proliferated cells is reacted in accordance with, for example, an enzyme labeling immunoassay with the nucleocapsid protein obtained by the above-described method, thereby selecting an anti-SARS-CoV-2 antibody production strain. When an animal other than mice is immunized, a lymphatic tissue is extracted from the animal immunized with the immunizing antigen, and an antibody gene is amplified to prepare an antibody library. Thereafter, the antibody library is introduced into Escherichia coli, and positive clones are selected.

Alternatively, RNA is extracted from a hybridoma or a B cell, and cDNA of the heavy chain and the light chain of the antibody gene is synthesized based on the extracted RNA. Here, the cDNA synthesized may express a part of an immunoglobulin molecule such as a CDR region. Thereafter, a heavy chain expression clone and a light chain expression clone are co-introduced into a mammal cell, a heavy chain and a light chain are co-expressed in the cell, and the culture supernatant is collected to prepare an antibody.

The antibody of the present invention may be any of an antibody *per se,* and an antibody fragment and a modified antibody against the nucleocapsid protein of SARS-CoV-2, which are substantially equivalent in performance to the antibody *per se.* The antibody fragment may be an Fab fragment, an Fab' fragment, an F(ab')₂ fragment, an scFv fragment or the like, or a part of immunoglobulin molecule such as a V region, a VH region or a CDR region as long as it specifically recognizes the nucleocapsid protein of SARS-CoV-2 at the same or equivalent level as the antibody of the present invention. The modified antibody may be one that specifically recognizes the nucleocapsid protein of SARS-CoV-2 at a level equivalent to the antibody of the present invention, and specific examples thereof include animal species-altered antibodies such as chimeric or humanized antibodies, subtype-altered antibodies, isotype-altered antibodies, antibodies that are recombined or synthesized in a genetic engineering manner, and antibodies that are recombined in a genetic engineering manner so that they can bind to both of two types of antigens or two different types of antigen determinant groups.

The antibody of the present invention can recognize a nucleocapsid protein consisting of 419 amino acids forming the full length of SARS-CoV-2 (SEQ ID NO: 1). The antibody of the present invention is only required to be capable of recognizing a nucleocapsid protein of SARS-CoV-2, and may recognize a N-terminal domain (NTD) consisting of the 41st to 186th amino acids of the nucleocapsid protein. Further, the antibody of the present invention may recognize a region consisting of the 247th to 419th amino acids which is a C-terminal domain (CTD).

The antibody that recognizes the N-terminal domain (NTD) in the present invention is (1) an antibody in which CDR1, CDR2 and CDR3 of a heavy chain comprise amino acid sequences set forth as SEQ ID NOS: 2, 3 and 4, respectively, and CDR1, CDR2 and CDR3 of a light chain comprise amino acid sequences set forth as SEQ ID NOS: 5, 6 and 7, respectively, (2) an antibody in which CDR1, CDR2 and CDR3 of a heavy chain comprise amino acid sequences set forth as SEQ ID NOS: 8, 9 and 10, respectively, and CDR1, CDR2 and CDR3 of a light chain comprise amino acid sequences set forth as SEQ ID NOS: 11, 12 and 13, respectively, (3) an antibody in which CDR1, CDR2 and CDR3 of a heavy chain comprise amino acid sequences set forth as SEQ ID NOS: 14, 15 and 16, respectively, and CDR1, CDR2 and CDR3 of a light chain comprise amino acid sequences set forth as SEQ ID NOS: 17, 18 and 19, respectively, (4) an antibody in which CDR1, CDR2 and CDR3 of a heavy chain comprise amino acid sequences set forth as SEQ ID NOS: 20, 21 and 22, respectively, and CDR1, CDR2 and CDR3 of a light chain comprise amino acid sequences set forth as SEQ ID NOS: 23, 24 and 25, respectively, (5) an antibody in which CDR1, CDR2 and CDR3 of a heavy chain comprise amino acid sequences set forth as SEQ ID NOS: 26, 27 and 28, respectively, and CDR1, CDR2 and CDR3 of a light chain comprise amino acid sequences set forth as SEQ ID NOS: 29, 30 and 31, respectively, (6) an antibody in which CDR1, CDR2 and CDR3 of a heavy chain comprise amino acid sequences set forth as SEQ ID NOS: 32, 33 and 34, respectively, and CDR1, CDR2 and CDR3 of a light chain comprise amino acid sequences set forth as SEQ ID NOS: 35, 36 and 37, respectively, (7) an antibody in which CDR1, CDR2 and CDR3 of a heavy chain comprise amino acid sequences set forth as SEQ ID NOS: 38, 39 and 40, respectively, and CDR1, CDR2 and CDR3 of a light chain comprise amino acid sequences set forth as SEQ ID NOS: 41, 42 and 43, respectively, (8) an antibody in which CDR1, CDR2 and CDR3 of a heavy chain comprise amino acid sequences set forth as SEQ ID NOS: 44, 45 and 46, respectively, and CDR1, CDR2 and CDR3 of a light chain comprise amino acid sequences set forth as SEQ ID NOS: 47, 48 and 49, respectively, (9) an antibody in which CDR1, CDR2 and CDR3 of a heavy chain comprise amino acid sequences set forth as SEQ ID NOS: 50, 51 and 52, respectively, and CDR1, CDR2 and CDR3 of a light chain comprise amino acid sequences set forth as SEQ ID NOS: 53, 54 and 55, respectively, (10) an antibody in which CDR1, CDR2 and CDR3 of a heavy chain comprise amino acid sequences set forth as SEQ ID NOS: 56, 57 and 58, respectively, and CDR1, CDR2 and CDR3 of a light chain comprise amino acid sequences set forth as SEQ ID NOS: 59, 60 and 61, respectively, (11) an antibody in which CDR1, CDR2 and CDR3 of a heavy chain comprise amino acid sequences set forth as SEQ ID NOS: 62, 63 and 64, respectively, and CDR1, CDR2 and CDR3 of a light chain comprise amino acid sequences set forth as SEQ ID NOS: 65, 66 and 67, respectively, (13) an antibody in which CDR1, CDR2 and CDR3 of a heavy chain comprise amino acid sequences set forth as SEQ ID NOS: 74, 75 and 76, respectively, and CDR1, CDR2 and CDR3 of a light chain comprise amino acid sequences set forth as SEQ ID NOS: 77, 78 and 79, respectively, (17) an antibody in which CDR1, CDR2 and CDR3 of a heavy chain comprise amino acid sequences set forth as SEQ ID NOS: 98, 99 and 100, respectively, and CDR1, CDR2 and CDR3 of a light chain comprise amino acid sequences set forth as SEQ ID NOS: 101, 102 and 103, respectively, (18) an antibody in which CDR1, CDR2 and CDR3 of a heavy chain comprise amino acid sequences set forth as SEQ ID NOS: 104, 105 and 106, respectively, and CDR1, CDR2 and CDR3 of a light chain comprise amino acid sequences set forth as SEQ ID NOS: 107, 108 and 109, respectively, or (20) an antibody in which CDR1, CDR2 and CDR3 of a heavy chain comprise amino acid sequences set forth as SEQ ID NOS: 116, 117 and 118, respectively, and CDR1, CDR2 and CDR3 of a light chain comprise amino acid sequences set forth as SEQ ID NOS: 119, 120 and 121, respectively.

The antibody that recognizes the C-terminal domain (CTD) in the present invention is (12) an antibody in which CDR1, CDR2 and CDR3 of a heavy chain comprise amino acid sequences set forth as SEQ ID NOS: 68, 69 and 70, respectively, and CDR1, CDR2 and CDR3 of a light chain comprise amino acid sequences set forth as SEQ ID NOS: 71, 72 and 73, respectively, (14) an antibody in which CDR1, CDR2 and CDR3 of a heavy chain comprise amino acid sequences set forth as SEQ ID NOS: 80, 81 and 82, respectively, and CDR1, CDR2 and CDR3 of a light chain comprise amino acid sequences set forth as SEQ ID NOS: 83, 84 and 85, respectively, (16) an antibody in which CDR1, CDR2 and CDR3 of a heavy chain comprise amino acid sequences set forth as SEQ ID NOS: 92, 93 and 94, respectively, and CDR1, CDR2 and CDR3 of a light chain comprise amino acid sequences set forth as SEQ ID NOS: 95, 96 and 97, respectively, or (19) an antibody in which CDR1, CDR2 and CDR3 of a heavy chain comprise amino acid sequences set forth as SEQ ID NOS: 110, 111 and 112, respectively, and CDR1, CDR2 and CDR3 of a light chain comprise amino acid sequences set forth as SEQ ID NOS: 113, 114 and 115, respectively.

The antibody that recognizes a region located between the N-terminal domain and the C-terminal domain in the present invention is (15) an antibody in which CDR1, CDR2 and CDR3 of a heavy chain comprise amino acid sequences set forth as SEQ ID NOS: 86, 87 and 88, respectively, and CDR1, CDR2 and CDR3 of a light chain comprise amino acid sequences set forth as SEQ ID NOS: 89, 90 and 91, respectively.

When the first antibody is an antibody that recognizes the N-terminal domain, the second antibody may be any of an antibody that recognizes the N-terminal domain, an antibody that recognizes the C-terminal domain, and an antibody that recognizes the region located between the N-terminal domain and the C-terminal domain.

When the first antibody is an antibody that recognizes the C-terminal domain, the second antibody may be any of an antibody that recognizes the N-terminal domain, an antibody that recognizes the C-terminal domain, and an antibody that recognizes the region located between the N-terminal domain and the C-terminal domain.

When the first antibody is an antibody that recognizes the region located between the N-terminal domain and the C-terminal domain, the second antibody may be any of an antibody that recognizes the N-terminal domain, an antibody that recognizes the C-terminal domain, and an antibody that recognizes the region located between the N-terminal domain and the C-terminal domain.

### Detection method

The present invention also provides a method for detecting the presence of SARS-CoV-2 in a test sample by an immunoassay using an antibody or a fragment thereof that specifically reacts with the nucleocapsid protein of SARS-CoV-2. Preferably, the immunoassay comprises contacting a test sample with the antibody or fragment thereof according to the present invention. In the detection method of the present invention, when the test sample contains SARS-CoV-2, a complex of the antibody or fragment thereof and SARS-CoV-2 is formed in the aforementioned contacting, and for example, by quantitatively or qualitatively measuring the complex, the SARS-CoV-2 nucleocapsid protein, and hence SARS-CoV-2 can be detected.

The detection of the complex in the detection method of the present invention can be performed using any of common immunoassays. Specific examples of the immunoassay include an enzyme immunoassay method (ELISA method or EIA method), a fluorescent immunoassay method (FIA), a radioimmunoassay method (RIA), a luminescent immunoassay method (LIA), an enzyme antibody method, a fluorescence antibody method, an immunochromatography method (immunochromato method), a flow-through method, an immunoturbidimetric method, a latex turbidimetric method, a latex agglutination reaction measurement method, a hemagglutination reaction method, and a particle agglutination reaction method. As the immunoassay for the SARS-CoV-2 nucleocapsid protein in the present invention, the ELISA method, the immunochromatography method and the flow-through method, which are sandwich methods using two antibodies or fragments thereof, are preferable. The ELISA method requires a determination time, but is excellent in sensitivity and quantitative performance, and enables multiple analytes to be treated at the same time. The immunochromatography method is inferior in sensitivity and quantitative performance to the ELISA method, but enables rapid and simple detection. The flow-through method involves complicated operations compared to the immunochromatography method, but is equivalent in sensitivity and quantitative performance to the ELISA method, and enables rapid detection.

Preferably, two antibodies or fragments thereof for use in the sandwich method recognize different epitopes, respectively, for avoiding a competitive reaction between the antibodies and exhibiting high reactivity.

The immunochromatography method of the present invention is typically an immunochromatography method involving: providing a membrane carrier having a capture portion formed by previously fixing a first antibody at a predetermined position, the first antibody being capable of undergoing an antigen-antibody reaction at a first antigen determinant group of an antigen; and chromatographically developing a mixed liquid of a predetermined amount of a test sample and a second antibody that is capable of undergoing an antigen-antibody reaction at a second antigen determinant group of the antigen and is labeled, toward the capture portion in the membrane carrier; whereby a complex of the antigen and the second antibody is captured at the capture portion if the test sample contains the antigen. The method can be carried out using the antibodies or fragments thereof according to the present invention as the first antibody and the second antibody.

The test sample in the present invention is not limited, and examples thereof include biological samples in which SARS-CoV-2 may be present, such as nasal aspirates, nasal swabs, nasopharynx swabs, pharynx swabs, sputum, saliva and bronchoalveolar lavage fluid. The method for collecting such a test sample is not limited, and the test sample is collected by a known method. For example, in the case of the nasal swab, nasopharynx swab and pharynx swab, a method using a cotton swab is often used. For example, in the case of the immunochromatography method, the collected test sample may be directly added to the membrane carrier, or may be diluted with an appropriate diluting liquid such as a developing solvent, followed by addition to the membrane carrier. Further, the collected test sample may be filtered using a filter for removing sticky materials and solid materials derived from biological components contained in the test sample, followed by addition to the membrane carrier.

When a test sample contaminated with blood is used, and in particular, a color labeling substance such as gold colloid is used as a labeling substance of a labeled antibody, it is preferable to dispose a blood cell capturing member on a member for adding sample. This prevents development of erythrocytes in the membrane carrier, so that accumulation of the color labeling substance at the capture portion of the membrane carrier is easily confirmed. As the blood cell capturing membrane member, a carboxymethyl cellulose membrane is used, and specifically, ion exchange filter paper CM (trade name) sold by ADVANTEC TOYO KAISHA, LTD., ion exchange cellulose paper sold by Whatman Japan K.K., and the like may be used.

### Detection kit

The detection method of the present invention may be carried out in a form based on the immunoassay, and is preferably carried out in a form based on the immunochromatography method that can be carried out rapidly and simply. The immunochromatography method can be easily carried out in line with the configuration of a known immunochromatography test strip.

In general, the immunochromatographic test strip comprises a first antibody capable of undergoing an antigen-antibody reaction at a first antigen determinant group of the nucleocapsid protein of SARS-CoV-2 which is an antigen, a second antibody which is capable of undergoing an antigen-antibody reaction at a second antigen determinant group of the antigen and is labeled, and a membrane carrier, the first antibody being previously fixed at a predetermined position on the membrane carrier to form a capture portion, the second antibody being disposed at a distance from the capture portion so as to be chromatographically developable in the membrane carrier. Each of the first antibody and the second antibody may be either a polyclonal antibody or a monoclonal antibody as described above, and it is preferable that one of the antibodies is a monoclonal antibody, and it is more preferable that both the antibodies are monoclonal antibodies. Normally, the first antigen determinant group and the second antigen determinant group are different in both position on the antigen and structure, and the first antibody and the second antibody are used in "hetero" combination.

A large number of nucleocapsid proteins are present together with RNA in the virus, and for avoiding a competitive reaction between antibodies and achieving high reactivity, the first antibody and the second antibody are preferably antibodies that recognize different antigen determinant groups present in the SARS-CoV-2 nucleocapsid protein.

An example of the immunochromatographic test strip is shown in Figure 1. Figure 1 is illustrative, and the shape and configuration of the strip, and the type and structure of the members used therein are not limited thereto. In Figure 1, reference numeral 1 denotes an adhesive sheet, reference numeral 2 denotes an impregnation member, reference numeral 3 denotes a membrane carrier, reference numeral 4 denotes an absorbing member, reference numeral 5 denotes a member for adding sample, reference numeral 31 denotes a capture portion, and reference numeral 32 denotes a control capture portion.

For example, for the membrane carrier 3, a long and thin strip-shaped nitrocellulose membrane filter 5 mm in width and 36 mm in length is used, and at a position of 7.5 mm from an end of the filter on the chromatographic development start point side, the first antibody is fixed to form the capture portion 31 for analyte. Further, the control capture portion 32 is provided at a position of 15 mm from an end of the membrane carrier 3 on the chromatographic development start point side, so that it is possible to confirm that chromatographic development of the test sample has been properly performed regardless of whether the analyte is present or not. Normally, a substance (excluding the analyte) immunologically specifically binding to the second antibody can be immobilized on the membrane carrier 3 to form the control capture portion 32. For example, when an antibody of mouse origin is used as the second antibody, the control capture portion 32 can be formed by using an antibody against the mouse antibody.

As the membrane carrier 3, a nitrocellulose membrane filter is used, but the membrane carrier 3 is only required to enable chromatographic development of the test sample, fixation of the first antibody, and formation of the capture portion 31 and the control capture portion 32, and other cellulose membranes, nylon membranes, glass fiber membranes and the like may be used.

The impregnation member 2 comprises a member impregnated with a second antibody that undergoes an antigen-antibody reaction with the antigen at a second antigen determinant group positioned at a portion different from a first antigen determinant group to which the first antibody binds, and the second antibody is previously labeled with an appropriated labeling substance.

For example, in the illustrated example, a glass fiber nonwoven fabric of 5 mm in width and 15 mm in length is used as the impregnation member 2, but the impregnation member is not limited thereto, and cellulose fabrics (filter paper, nitrocellulose membranes and the like), porous plastic fabrics of polyethylene, polypropylene, etc., and the like may also be used for the impregnation member 2.

The labeling substance for the second antibody may be any substance as long as it is usable, and examples thereof include color labeling substances, enzyme labeling substances, fluorescence labeling substances, and radiation labeling substances. Of these, color labeling substances are preferably used because a determination can be made rapidly and simply by visually observing a change in color at the capture portion 31.

Examples of the color labeling substance include metal colloids such as gold colloid and platinum colloid, composite metal colloids thereof, and latexes such as synthetic latex such as polystyrene latex and natural rubber latex which are colored with red and blue pigments. Of these, metal colloids such as gold colloid are particularly preferable.

The impregnation member 2 can be prepared by impregnating a member such as a glass fiber nonwoven fabric with a suspension liquid of a second antibody labeled with the labeling substance, followed by drying.

In Figure 1, the chromatographic development start point side is defined as the "upstream", and the direction in which the developing solvent flows (on the absorbing member 4 side) is defined as the downstream. The membrane carrier 3 is attached to a middle part of the adhesive sheet 1, and the downstream end of the impregnation member 2 is superposed on the upstream end of the membrane carrier 3 to connect the impregnation member 2 to the membrane carrier 3, and the upstream part of the impregnation member 2 is attached to the adhesive sheet 1. In this way, the immunochromatographic test strip of the present invention can be prepared.

The upstream end of the absorbing member 4 is superposed on the upper surface of the downstream part of the membrane carrier 3 to connect the absorbing member 4 to the membrane carrier 3, and the downstream part of the absorbing member 4 is attached to the adhesive sheet 1. Further, an arrangement is made to cover the upper surface of the impregnation member 2 with the downstream part of the member 5 for adding sample, and the upstream part of the member 5 for adding sample is attached to the adhesive sheet 1.

The absorbing member 4 may be any member capable of rapidly absorbing liquid, and holding the liquid, and examples thereof include cotton cloth, filter paper, and porous plastic nonwoven fabric of polyethylene or polypropylene, among which filter paper is most suitable.

As the member 5 for adding sample, sheets or films of porous synthetic resins such as porous polyethylene and porous polypropylene, and cellulose paper or woven or nonwoven fabric such as filter paper and cotton cloth may be used.

Further, the immunochromatographic test strip of Figure 1 can be provided in a state of being contained in an appropriate plastic case with a test sample injection part and a determination part which are respectively opened above the member 5 for adding sample and the capture portion 31. It is preferable that the immunochromatographic test strip is provided in a state of being contained in the plastic case for the purpose of preventing secondary infection of users.

It is preferable that the immunochromatography kit of the present invention comprise a developing solvent for developing the analyte, in addition to the immunochromatographic test strip.

The developing solvent can also be used as a liquid for diluting the analyte, and typically contains water as a solvent to which one kind or two or more kinds selected from a buffer solution, an inorganic salt, an organic salt, a surfactant, a protein, a polymer compound, a polyanion, an antibacterial agent, a chelating agent and the like may be added.

The surfactant, the protein, the polymer compound and the polyanion may be added for promoting the antigen-antibody reaction or suppressing a nonspecific reaction.

Particularly, in carrying out the present invention, it is preferable to incorporate a nonionic surfactant in the developing solvent as a means effective for dissolving an envelope comprising a lipid bilayer which forms a SARS-CoV-2 particle, releasing the internal nucleocapsid protein, and exposing an antigen-recognition site for the anti-SARS-CoV-2 antibody. Examples of the nonionic surfactant include polyethylene glycol mono-p-isooctylphenyl ether, polyoxyethylene sorbitan monolaurate, and Brij (trademark) 35 (trade name), and one or more thereof may be added.

Now, a test sample comprising a biological sample and the like is mixed with a developing solvent as necessary to obtain a mixed liquid that can be chromatographically developed, and the mixed liquid is then added to the member 5 for adding sample of the immunochromatographic test strip shown in Figure 1. The mixed liquid passes through the member 5 for adding sample, and is mixed with the labeled second antibody in the impregnation member 2. At this time, if the analyte is present in the mixed liquid, a complex of the analyte and the second antibody is formed by an antigen-antibody reaction.

The complex is chromatographically developed in the membrane carrier 3 to reach the capture portion 31, and undergoes the antigen-antibody reaction with the first antibody fixed to the portion, thereby being captured. When a color labeling substance such as gold colloid is used as a labeling substance, the capture portion 31 develops a color due to accumulation of the color labeling substance, so that immediately, the analyte can be qualitatively measured. Further, the color strength of the color substance accumulating at the capture portion 31 of the immunochromatographic test strip is optically read by an immunochromatography reader to quantify the color strength, so that quantitative measurement can be performed.

If chromatographic development is properly performed, the second antibody that has not undergone the antigen-antibody reaction with the analyte is captured by an antibody against the second antibody which is fixed to the control capture portion 32, and the control capture portion 32 develops a color similarly to the capture portion 31, so that it is confirmed that chromatographic development has been properly performed. On the other hand, if the control capture portion 32 does not develop a color, it is confirmed that there has arisen a problem that the second antibody is not developed, for example.

The second antibody is impregnated into the impregnation member 2 and disposed on the membrane carrier 3 in the illustrated immunochromatographic test strip, but is not necessarily in the illustrated form as long as the second antibody is provided so as to be developable in the membrane carrier 3 at a distance from the capture portion 31. For example, it is possible that the second antibody is previously contained in a container such as a tube provided for mixing with the test sample and the developing solvent, and packaged together with a test strip comprising the membrane carrier 3 to which the first antibody is previously fixed.

### Examples

The present invention will be described in further detail by way of the following Examples, which should not be construed as limiting the present invention.

### Example 1: Expression and purification of recombinant nucleocapsid protein

A plasmid with a nucleocapsid protein gene of SARS-CoV-2, specifically pcMV3-2019-nCov-NP-His (Sino bio), was introduced into E. coli DH5α Competent Cells (Takara Bio Inc.), and cultured overnight in Terrific broth containing kanamycin, thereby amplifying the plasmid. The amplified plasmid was transfected into 293T cells, and the cells were collected and lysed after 7 days. The cell lysate was subjected to ultrasonic treatment, and then purified using an affinity column. The purified protein was dialyzed against phosphate buffered saline (hereinafter, referred to as PBS) to obtain a desired recombinant nucleocapsid protein.

### Example 2: Production of monoclonal antibody against recombinant nucleocapsid protein

SARS-CoV-2 coronavirus nucleocapsid protein NP (manufactured by CUSABIO LLC.) (hereinafter, referred to as SARS-CoV-2 recombinant NP) and the recombinant nucleocapsid protein prepared in Example 1 were used as nucleocapsid proteins of SARS-CoV-2. With each recombinant protein as an immunizing antigen, a monoclonal antibody against the nucleocapsid protein of SARS-CoV-2 was produced. The antigen applied for immunization of mice was prepared by previously mixing the recombinant protein and an equal amount of an adjuvant (Becton, Dickinson and Company Japan). The mixture of a recombinant protein and an adjuvant was applied for immunization of mice (BALB/c, 6 weeks old, Japan SLC. Inc.) multiple times so that the antigen amount reached 100 µg, and the spleen cells of the mice were used for cell fusion. For the cell fusion, Sp2/0-Ag14 cells which are myeloma cells of mice were used (Shulman et al., 1987). The cell fusion was performed by mixing the spleen cells and the Sp2/0-Ag14 cells of mice, and adding a polyethylene glycol solution (Sigma). The hybridomas were cultured in HAT-RPMI (serum-containing RPMI containing 0.1 mM sodium hypoxanthine, 0.4 µM aminopterin and 0.1 mM thymidine), and antibody production in the culture supernatant was confirmed by an enzyme-linked immunoassay method (ELISA). Cells positive in antibody production were cultured in HT-RPMI (serum-containing RPMI containing 0.1 mM sodium hypoxanthine and 0.1 mM thymidine), and further cultured in a serum-containing medium.

### Example 3: Preparation of monoclonal antibody

The cells cloned in Example 2 were inoculated into the abdominal cavity of mice (BALB/c, retire, Charles River Laboratories Japan, Inc.) previously inoculated with 2,6,10,14-tetramethylpentadecane (Kishida Chemical Co., Ltd.). Thereafter, the peritoneal fluid was collected from the mice, and applied to a protein G column to purify monoclonal antibodies.

Finally, 83 clones of cells producing monoclonal antibodies against the nucleocapsid protein of SARS-CoV-2 were obtained.

### Example 4: Analysis of amino acids of anti-SARS-CoV-2 antibody

The complementarity determining regions (CDRs) of 10 clones selected from the anti-SARS-CoV-2 antibodies obtained in Example 3 were analyzed. The monoclonal antibody producing cells prepared in Example 2 were cultured, and RNA was purified from numbers of cells predetermined. With the purified RNA as a template, cDNA was synthesized using an oligo dT primer and SuperScript (trademark) III reverse transcriptase (ThermoFisher Scientific). Thereafter, using a 3' reverse primer for a sequence specific to a constant region of an antibody, the sequence of a variable region was amplified, and sequence analysis was then performed. The sequence of the light chain was inserted into the T vector, and then transformed, a T plasmid was purified, and sequence analysis was performed. The DNA sequence decoded by the sequence analysis was analyzed using three programs, IMGT/V-QUEST, VBASE2 and IGBLAST, and consistency in identified CDR was confirmed. Table 1 shows the results of CDR sequence analysis. It is obvious to those skilled in the art that in the present invention, one or several amino acids in the CDR sequence of each antibody shown in Table 1 may be deleted, substituted or added as long as the SARS-CoV-2 nucleocapsid protein is recognized at the same or equivalent level.

**Table 1**

| CDR sequence of heavy chain and light chain of obtained anti-SARS coronavirus-2 antibody | | | |
|---|---|---|---|
| Antibody name | CDR sequence | Heavy chain | Light chain |
| TAU001 | 1 | GYIFTDFI (SEQ ID NO: 2) | QNLLDSDGKTY (SEQ ID NO: 5) |
| | 2 | ISTYYGDA (SEQ ID NO: 3) | LVS (SEQ ID NO: 6) |
| | 3 | ARGGDYDEFYAMDH (SEQ ID NO: 4) | CQGTHFPQT (SEQ ID NO: 7) |
| TAU002 | 1 | GYTFTDYS (SEQ ID NO: 8) | ENIYST (SEQ ID NO: 11) |
| | 2 | INTETNES (SEQ ID NO: 9) | AAT (SEQ ID NO: 12) |
| | 3 | GSPYYYGNSYDWYFDV (SEQ ID NO: 10) | QHFWGFPWT (SEQ ID NO: 13) |
| TAU003 | 1 | GYTLTDYS (SEQ ID NO: 14) | ENIYSN (SEQ ID NO: 17) |
| | 2 | VNTETADP (SEQ ID NO: 15) | LAT (SEQ ID NO: 18) |
| | 3 | ARGYYYGNTHDY (SEQ ID NO: 16) | QHFWGTPYT (SEQ ID NO: 19) |
| TAU004 | 1 | GYSFTDYT (SEQ ID NO: 20) | QSLLYSSTQKNY (SEQ ID NO: 23) |
| | 2 | INPNHGGT (SEQ ID NO: 21) | WAS (SEQ ID NO: 24) |
| | 3 | ARSGNFITTVGGLLDY (SEQ ID NO: 22) | QQYYSYPYT (SEQ ID NO: 25) |
| TAU005 | 1 | GYTFTNYG (SEQ ID NO: 26) | ENIYSS (SEQ ID NO: 29) |
| | 2 | INTYTGEP (SEQ ID NO: 27) | TAT (SEQ ID NO: 30) |
| | 3 | ARNYANYNYFDY (SEQ ID NO: 28) | QHFWGTPWT (SEQ ID NO: 31) |
| TAU006 | 1 | GYTFTDYG (SEQ ID NO: 32) | ENIYGN (SEQ ID NO: 35) |
| | 2 | INTHTGEP (SEQ ID NO: 33) | TAT (SEQ ID NO: 36) |
| | 3 | ARRGLYYDYDGRGFVY (SEQ ID NO: 34) | QHFWGTPWT (SEQ ID NO: 37) |
| TAU007 | 1 | GYPFTNYG (SEQ ID NO: 38) | QSVSTSTYTY (SEQ ID NO: 41) |
| | 2 | INTNTGEP (SEQ ID NO: 39) | YAS (SEQ ID NO: 42) |
| | 3 | ARDDNFWFAY (SEQ ID NO: 40) | QHTWDIPYT (SEQ ID NO: 43) |
| TAU008 | 1 | GYTFTNYG (SEQ ID NO: 44) | ENIYSN (SEQ ID NO: 47) |
| | 2 | INTDTGEP (SEQ ID NO: 45) | VAT (SEQ ID NO: 48) |
| | 3 | ASSAYYGNWFAY (SEQ ID NO: 46) | QHFWGTPWT (SEQ ID NO: 49) |
| TAU009 | 1 | GYTFTDYG (SEQ ID NO: 50) | ENVYGN (SEQ ID NO: 53) |
| | 2 | INTDTGEP (SEQ ID NO: 51) | VAT (SEQ ID NO: 54) |
| | 3 | ASSAYHGNWFLY (SEQ ID NO: 52) | QHFWGTPWT (SEQ ID NO: 55) |
| TAU010 | 1 | GYTFTNYG (SEQ ID NO: 56) | QSISTSDYIY (SEQ ID NO: 59) |
| | 2 | INTNTGEP (SEQ ID NO: 57) | YAS (SEQ ID NO: 60) |
| | 3 | ARDGNFWFAY (SEQ ID NO: 58) | QHSWEIPYT (SEQ ID NO: 61) |

### Example 5: Reactivity test on anti-SARS-CoV-2 antibody

The anti-SARS-CoV-2 antibody obtained in Example 3 was tested for reactivity. For evaluating the reactivity of the antibody, microplates were prepared on which a recombinant protein, SARS-CoV-2 recombinant NP, human coronavirus 229E (VR-740 strain obtained from ATCC) and human coronavirus OC43 (VR-1558 strain obtained from ATCC) were immobilized at a predetermined concentration. To each of the immobilized microplates, each of the antibodies prepared in Example 3 was added and reacted at room temperature for 1 hour. After completion of the reaction, the solution in the wells was removed by suction, an anti-mouse antibody labeled with horse radish peroxidase was then added, and the mixture was reacted for 1 hour. Thereafter, a 3,3',5,5'-tetramethylbendine solution was added as a chromogenic substrate, the mixture was reacted, and the reaction was quenched with 2 N sulfuric acid. Using a microplate reader, the absorbance at 450 nm in each well of the microplate after quenching of the reaction was measured. Table 2 shows the results.

**Table 2**

| Test for reactivity of anti-SARS coronavirus-2 antibody | | | |
|---|---|---|---|
| Antibody name | SARS-CoV-2 recombinant NP | Human coronavirus 229E | Human coronavirus OC43 |
| Blank | 0.053 | 0.014 | 0.010 |
| TAU001 | 1.914 | 0.026 | 0.030 |
| TAU002 | 2.143 | 0.009 | 0.018 |
| TAU003 | 2.154 | 0.025 | 0.031 |
| TAU004 | 1.813 | 0.014 | 0.014 |
| TAU005 | 1.791 | 0.014 | 0.014 |
| TAU006 | 1.821 | 0.012 | 0.013 |
| TAU007 | 2.766 | 0.015 | 0.016 |
| TAU008 | 2.650 | 0.004 | 0.005 |
| TAU009 | 2.996 | 0.006 | 0.004 |
| TAU010 | 2.006 | 0.009 | 0.011 |

From Table 2, it was confirmed that the anti-SARS-CoV-2 antibodies prepared in Example 3 exhibited high reactivity to the recombinant protein, SARS-CoV-2 recombinant NP, and did not undergo cross reaction with human coronavirus 229E and OC43 as a negative control. Therefore, the obtained anti-SARS-CoV-2 antibodies were shown to specifically bind to the nucleocapsid protein of SARS-CoV-2.

Further, a reactivity test was conducted by the above-described method using a recombinant nucleocapsid protein consisting of the 41st to 186th amino acids positioned at the N-terminus of the SARS-CoV-2 nucleocapsid protein set forth as SEQ ID NO: 1 (CUSABIO LLC.), and a recombinant nucleocapsid protein consisting of the 220th to 419th amino acids positioned at the C-terminus of SARS-CoV-2 nucleocapsid protein (CUSABIO LLC.). Table 3 shows the results.

**Table 3**

| Antibody name | SARS-CoV-2 recombinant NP amino acids 41-186 | SARS-CoV-2 recombinant NP amino acids 220-419 |
|---|---|---|
| Blank | 0.017 | 0.007 |
| TAU001 | 1.369 | 0.004 |
| TAU002 | 1.859 | 0.003 |
| TAU003 | 2.148 | 0.020 |
| TAU004 | 1.455 | 0.002 |
| TAU005 | 1.085 | 0.003 |
| TAU006 | 2.010 | 0.012 |
| TAU007 | 1.371 | 0.008 |
| TAU008 | 1.401 | 0.010 |
| TAU009 | 2.224 | 0.001 |
| TAU010 | 0.843 | 0.005 |

From Table 3, the antibodies prepared in Example 3 were confirmed to react with the recombinant nucleocapsid protein consisting of the 41st to 186th amino acids positioned at the N-terminus. Thus, the anti-SARS-CoV-2 antibodies obtained were shown to recognize an epitope positioned in a N-terminal region of the SARS-CoV-2 nucleocapsid protein, in particular, in a region consisting of 41st to 186th amino acids from the N-terminus of the SARS-CoV-2 nucleocapsid protein.

### Example 6: Calculation of dissociation rate constant of antibody against anti-SARS-CoV-2

The dissociation constant (Kd) against the SARS-CoV-2 nucleocapsid protein of each of the antibodies prepared in Example 3 was calculated. The Kd value was calculated by a biolayer interference method using BLItz System (trade name) manufactured by ForteBio Inc. A biosensor chip was used on which an anti-mouse IgG Fv antibody was immobilized.

As a measurement setup, the biosensor chip was immersed in 200 µL of a buffer solution for 10 minutes to make the sensor chip compatible with water.

For measuring the dissociation constant of the antibody, the biosensor chip was washed with a buffer solution for 30 seconds to perform calibration to an initial value. Then, the solution of the antibody obtained in Example 3 was adjusted to 100 nM, and 4 µL of the antibody solution was reacted with the biosensor chip to immobilize the anti-SARS-CoV-2 antibody on the surface of the biosensor chip over 120 seconds, followed by washing with the buffer solution for 30 seconds for removing unbound antibodies. Subsequently, 4 µL of SARS-CoV-2 recombinant NP adjusted to 100 nM was allowed to interact for 120 seconds with the anti-SARS-CoV-2 antibody immobilized on the biosensor chip surface, thereby carrying out an antigen-antibody reaction, and the reaction product was then washed with the buffer solution for 120 seconds to measure the binding ability of the antibody. After completion of measurement, the dissociation constant was calculated. Table 4 shows the calculated binding rate constants, dissociation rate constants and dissociation constants of each antibody.

**Table 4**

| Antibody name | Binding rate constant (1/Ms) | Dissociation rate constant (1/s) | Dissociation constant (M) |
|---|---|---|---|
| TAU001 | 1.1 × 10⁶ | 4.3 × 10⁻⁵ | 3.8 × 10⁻¹¹ |
| TAU002 | 1.1 × 10⁶ | 1.1 × 10⁻³ | 9.9 × 10⁻¹⁰ |
| TAU003 | 7.1 × 10⁵ | 9.7 × 10⁻⁴ | 1.4 × 10⁻⁹ |
| TAU004 | 4.9 × 10⁵ | 9.9 × 10⁻⁴ | 2.0 × 10⁻⁹ |
| TAU005 | 6.7 × 10⁵ | 1.2 × 10⁻³ | 1.7 × 10⁻⁹ |
| TAU006 | 8.1 × 10⁵ | 2.6 × 10⁻⁴ | 3.3 × 10⁻¹⁰ |
| TAU007 | 9.7 × 10⁵ | 7.9 × 10⁻⁵ | 8.1 × 10⁻¹¹ |
| TAU008 | 1.1 × 10⁶ | 2.3 × 10⁻⁴ | 2.2 × 10⁻¹⁰ |
| TAU009 | 1.1 × 10⁶ | 3.4 × 10⁻⁴ | 3.0 × 10⁻¹⁰ |
| TAU010 | 1.3 × 10⁶ | 4.5 × 10⁻⁴ | 3.4 × 10⁻¹⁰ |

From Table 4, it was shown that among the antibodies obtained here, the antibody having the lowest binding force had a dissociation constant of 2.0 nM, and the antibody having the highest binding force had a dissociation constant of 38 pM. This demonstrated that the antibodies obtained here had a dissociation constant of 2.0 nM or less against SARS-CoV-2-recombinant NP.

### Example 7: Preparation of immune cell sample against SARS-CoV-2 of rabbit origin

SARS-CoV-2 recombinant NP as a nucleocapsid protein of SARS-CoV-2 was used as an immunizing antigen, and blood was collected from rabbits to acquire immune cells. The antigen applied for immunization of rabbits was prepared by previously mixing the recombinant protein and an equal amount of an adjuvant (BD Japan). The mixture of SARS-CoV-2 recombinant NP and the adjuvant was applied for immunization of rabbits multiple times so that the antigen amount reached 500 µg. Blood was collected and the spleen was extracted. Only peripheral blood mononuclear cells (PBMCs) were separated and collected from the collected total blood by centrifugation to prepare a PBMC sample. The extracted spleen was cellularized by filtration to obtain a rabbit spleen sample.

### Example 8: Acquisition of anti-SARS-CoV-2 antibody gene of rabbit origin

For separating lymphocytes against SARS-CoV-2 from the rabbit spleen sample prepared in Example 7, a specific gravity centrifugal method was carried out with Lymphocyte Separation Medium 1077 (PromoCell GmbH) as a separation solution to fractionate a lymphocyte subset from the rabbit spleen sample, thereby preparing a lymphocyte sample. For fractionating B cells that react with SARS-CoV-2 recombinant NP from the PBMC sample and the lymphocyte sample, each sample and the fluorescently labeled SARS-CoV-2 nucleocapsid protein were reacted. B cells that reacted with the fluorescently labeled nucleocapsid protein were measured using a cell sorter (Sony Corporation), and cells confirmed to have reactivity were fractionated one by one to microplates by single cell sorting. The fractionated cells were subjected to single cell RT-PCR, and genes of monoclonal antibodies were obtained. In this way, antibody genes of 200 clones in total were obtained.

### Example 9: Gene sequence analysis of Fab antibody

The DNA sequences were analyzed for the heavy chains and the light chains of antibody genes from 10 clones, among the antibody genes obtained in Example 8. The cDNA encoding the variable region of each gene was introduced into a pRSET vector, and the resulting DNA sequence was analyzed by a Sanger method. Thereafter, based on the DNA sequence obtained, the amino acid sequence and the CDR of the Fab antibody were analyzed. With the DNA sequence analyzed, CDR was analyzed using three programs of IMGT/V-QUEST, VBASE2 and IGBLAST, and consistency in the identified CDR was confirmed. Table 5 shows the results of the CDR sequence analysis. It is obvious to those skilled in the art that in the present invention, one or several amino acids in the CDR sequence of each antibody shown in Table 5 may be deleted, substituted or added as long as the SARS-CoV-2 nucleocapsid protein is recognized at the same or equivalent level.

**Table 5**

| CDR sequence of heavy chain and light chain of anti-SARS-CoV-2 Fab antibody of rabbit origin | | | |
|---|---|---|---|
| Antibody name | CDR sequence | Heavy chain | Light chain |
| TN01 | 1 | GFDFSSQNY (SEQ ID NO: 62) | QSVFSDNY (SEQ ID NO: 65) |
| | 2 | IYTVSGET (SEQ ID NO: 63) | YAS (SEQ ID NO: 66) |
| | 3 | ARSPVKRIGVDMTRLDL (SEQ ID NO: 64) | QGYYSGYINV (SEQ ID NO: 67) |
| TN02 | 1 | GFDFSSDI (SEQ ID NO: 68) | ESVWNY (SEQ ID NO: 71) |
| | 2 | IVIDTTTT (SEQ ID NO: 69) | QAS (SEQ ID NO: 72) |
| | 3 | ARAFSL (SEQ ID NO: 70) | QGGYSGNIFP (SEQ ID NO: 73) |
| TN03 | 1 | GFDLNNYQY (SEQ ID NO: 74) | QSVYNN NL (SEQ ID NO: 77) |
| | 2 | IYTDSYIT (SEQ ID NO: 75) | KAS (SEQ ID NO: 78) |
| | 3 | ARDVYVGYAGYAYALNL (SEQ ID NO: 76) | LGGVDDDADPNT (SEQ ID NO: 79) |
| TN04 | 1 | GIDFTNYD (SEQ ID NO: 80) | SVYGNNY (SEQ ID NO: 83) |
| | 2 | ILAGSSGGP (SEQ ID NO: 81) | QAS (SEQ ID NO: 84) |
| | 3 | AGSGYRNSFYFGL (SEQ ID NO: 82) | QGTYWSDDWYNV (SEQ ID NO: 85) |
| TN05 | 1 | GFDFSTSA(SEQ ID NO: 86) | SVYDSQV(SEQ ID NO: 89) |
| | 2 | IDTGSGNT (SEQ ID NO: 87) | KAS (SEQ ID NO: 90) |
| | 3 | ARDFVL (SEQ ID NO: 88) | AGAYSGNWA(SEQ ID NO: 91) |
| TN06 | 1 | GFDFSGDIA(SEQ ID NO: 92) | ESVAVNY (SEQ ID NO: 95) |
| | 2 | IWDTTTT (SEQ ID NO: 93) | ETS (SEQ ID NO: 96) |
| | 3 | ARAFSL (SEQ ID NO: 94) | QGGYSGNDFP (SEQ ID NO: 97) |
| TN07 | 1 | GFDLSHFFY (SEQ ID NO: 98) | KSVYNNNY (SEQ ID NO: 101) |
| | 2 | IETAGGST (SEQ ID NO: 99) | GAS (SEQ ID NO: 102) |
| | 3 | ARSIYTGTYAAYAYALNL (SEQ ID NO: 100) | AGGYSTSSDNG (SEQ ID NO: 103) |
| TN08 | 1 | GFSFNNKHY (SEQ ID NO: 104) | EDIYSL (SEQ ID NO: 107) |
| | 2 | IYAGDGTT (SEQ ID NO: 105) | SAS (SEQ ID NO: 108) |
| | 3 | ARGDYYSYGYAYDI (SEQ ID NO: 106) | QCTYYSSSYA(SEQ ID NO: 109) |
| TN09 | 1 | GFDFSSDV (SEQ ID NO: 110) | ESWVKY (SEQ ID NO: 113) |
| | 2 | IVIDTTTT (SEQ ID NO: 111) | QAS (SEQ ID NO: 114) |
| | 3 | ARTFSL (SEQ ID NO: 112) | QGGYSGNIFP (SEQ ID NO: 115) |
| TN10 | 1 | GFSFSSSYY (SEQ ID NO: 116) | QNIYSN (SEQ ID NO: 119) |
| | 2 | IYAGSSGNT (SEQ ID NO: 117) | GTS (SEQ ID NO: 120) |
| | 3 | AGDEDYWDL (SEQ ID NO: 118) | QSAYYSSTSSWT (SEQ ID NO: 121) |

From Table 5, it was demonstrated that TN02, TN06 and TN09 differed in CDR sequences only by 1 or 2 amino acids and were similar in the heavy and the light chain CDRs, and therefore it was determined that these antibodies recognized an identical epitope of the antigen.

### Example 10: Preparation of Fab antibody in cell-free expression system and reactivity test

To each of antibody genes from 10 clones obtained in Example 8, a promoter sequence and a ribosome binding sequence for cell-free expression system were added to prepare a template. Each template was subjected to a cell-free translation-transcription coupling reaction by PUREfrex (trademark) 2.0 (GeneFrontier Corporation) to express the protein of an anti-SARS-CoV-2 Fab antibody. Here, for obtaining optimum conditions to form disulfide bond, DsbC Set (GeneFrontier Corporation) was added. In this way, the anti-SARS-CoV-2 Fab antibody was obtained. For the anti-SARS-CoV-2 Fab antibody obtained, the reactivity was evaluated. Here, an antibody having the H chain labeled with a HA tag was used. For evaluating the reactivity of the antibody, a microplate was prepared on which the SARS-CoV-2 recombinant NP obtained was immobilized at a predetermined concentration. The microplate was reacted with the HA tag-labeled anti-SARS-CoV-2 Fab antibody at a predetermined concentration, and then reacted with a horse radish peroxidase-labeled anti-HA tag antibody against the HA tag of the antibody. Thereafter, a 3,3',5,5'-tetramethylbendine solution was added as a chromogenic substrate, causing the reaction, and the reaction was quenched with 2 N sulfuric acid. Using a microplate reader, the absorbance was measured at 450 nm for each well of the microplate after quenching of the reaction.

The expression level of the anti-SARS-CoV-2 Fab antibody was evaluated. Specifically, the HA tag-labeled anti-SARS-CoV-2 Fab antibody obtained was immobilized on a microplate at a concentration 5 times the predetermined concentration set in the reactivity evaluation. The horse radish peroxidase-labeled anti-HA tag antibody against the HA tag added to the H chain of the immobilized antibody was reacted. Thereafter, a 3,3',5,5'-tetramethylbendine solution was added as a chromogenic substrate, causing reaction, and the reaction was quenched with 2 N sulfuric acid. Using a microplate reader, the absorbance at 450 nm in each well of the microplate after quenching of the reaction was measured. The reactivity per expression was calculated on percentage in terms of a reaction ratio. Since the concentrations of the antibody used differed between evaluation of the reactivity and evaluation of the expression, the reaction ratio was calculated with the measurement result corrected for antibody concentration.

**Table 6**

| Evaluation of reactivity of anti-SARS-CoV-2 Fab antibody of rabbit origin | | | |
|---|---|---|---|
| Antibody name | SARS-CoV-2 recombinant NP | Antibody expression level | Reaction ratio (%) |
| TN01 | 2.056 | 1.190 | 34.54 |
| TN02 | 0.605 | 0.469 | 25.83 |
| TN03 | 2.090 | 1.313 | 31.83 |
| TN04 | 1.596 | 1.275 | 25.03 |
| TN05 | 1.329 | 1.153 | 23.07 |
| TN06 | 1.719 | 1.384 | 24.85 |
| TN07 | 1.556 | 1.283 | 24.25 |
| TN08 | 0.490 | 0.353 | 27.71 |
| TN09 | 0.607 | 0.375 | 32.37 |
| TN10 | 1.058 | 0.660 | 32.04 |

From Table 6, some of the antibodies having high-absorbance against SARS-CoV-2 recombinant NP were confirmed to have high antibody expression and a low reaction ratio. On the other hand, some of the antibodies having low absorbance against the nucleocapsid protein were confirmed to have low antibody expression and a high reaction ratio. Actually, even an antibody having a low reaction ratio had high apparent reactivity if the antibody expression was high.

Among TN02, TN06 and TN09 determined to be similar to one another in Table 5, TN09 had the highest reaction ratio, and therefore, of the three antibodies, TN09 was selected, and used in the subsequent tests.

Further, a reactivity test was conducted using a recombinant nucleocapsid protein consisting of the 41st to 186th amino acids positioned at the N-terminus of the SARS-CoV-2 nucleocapsid protein set forth as SEQ ID NO: 1 (CUSABIO LLC.), and a recombinant nucleocapsid protein consisting of the 220th to 419th amino acids positioned at the C-terminus of SARS-CoV-2 nucleocapsid protein (CUSABIO LLC.). To a microplate on which each recombinant nucleocapsid protein was immobilized, each of the expressed Fab antibodies was added causing the reaction at room temperature for 1 hour. After completion of the reaction, the solution in wells was removed by suction, an anti-rabbit antibody labeled with horse radish peroxidase was then added, causing the reaction for 1 hour. Thereafter, a 3,3',5,5'-tetramethylbendine solution was added as a chromogenic substrate, causing the reaction, and the reaction was quenched with 2 N sulfuric acid. Using a microplate reader, after quenching of the reaction, the absorbance was measured for each well of the microplate at 450 nm. As shown in Table 5 of Example 9, the CDR sequences of TN02 and TN06 were similar to the CDR sequence of TN09, and therefore these three types of antibodies were suggested to bind to the same epitope. Thus, in the present test, the reaction region was evaluated only for TN09, which has the highest reaction ratio. Table 7 shows the results.

**Table 7**

| Evaluation of reaction region of SARS-CoV-2 antibody of rabbit origin | | |
|---|---|---|
| Antibody name | SARS-CoV-2 recombinant NP amino acids 41-186 | SARS-CoV-2 recombinant NP amino acids 220-419 |
| TN01 | 2.168 | 0.010 |
| TN03 | 2.346 | 0.004 |
| TN04 | 0.049 | 1.621 |
| TN05 | 0.005 | 0.034 |
| TN07 | 1.744 | 0.003 |
| TN08 | 1.697 | 0.005 |
| TN09 | 0.005 | 1.298 |
| TN10 | 2.225 | 0.028 |

From Table 7, it was shown that among the antibodies expressed with genes of rabbit origin, TN01, TN03, TN07, TN08 and TN10 reacted with a recombinant nucleocapsid protein consisting of the 41st to 186th amino acids positioned at the N-terminus, and therefore recognized an epitope positioned in a region of the 41st to 186th amino acids of the sequence set forth as SEQ ID NO: 1. On the other hand, it was shown that TN04 and TN09 reacted with a recombinant nucleocapsid protein consisting of the 220th to 419th amino acids positioned at the C-terminus, and therefore recognized an epitope positioned in a region of the 220th to 419th amino acids of the sequence set forth as SEQ ID NO: 1. TN05 did not react with either of the recombinant nucleocapsid proteins, and was therefore suggested to recognize an epitope positioned so as not to belong to either of the amino acid sequences, that is, an amino acid sequence consisting of the 187th to 219th amino acids.

### Example 11: Preparation of immunochromatographic test strip using anti-SARS-CoV-2 antibody

The anti-SARS-CoV-2 antibody purified in Example 3 and the anti-SARS-CoV-2 Fab antibody prepared in Example 10 were used for preparation of an immunochromatographic test strip.

### (1) Preparation of platinum-gold colloid particle solution

All glassware to be used was washed with aqua regia. In a flask, 390 mL of ultrapure water was boiled. To the boiled water, 30 mL of an aqueous chloroauric acid solution (1 g of gold per liter of aqueous solution, manufactured by KATAYAMA CHEMICAL INDUSTRIES Co., Ltd.) was added, followed by addition of 60 mL of a 1 wt% aqueous sodium citrate solution. Six minutes and forty-five seconds after the addition of the aqueous sodium citrate solution, 30 mL of an aqueous chloroplatinic acid solution (1 g of platinum per liter of aqueous solution, Wako Pure Chemical Industries Ltd.) was added. Five minutes thereafter, 60 mL of a 1 wt% aqueous sodium citrate solution was added, and the mixture was refluxed for four hours to obtain a platinum-gold colloid suspension liquid.

### (2) Preparation of platinum-gold colloid-labeled anti-SARS-CoV-2 antibody solution

Each of the anti-SARS-CoV-2 antibodies obtained in Example 3 was labeled with platinum-gold colloid by the following procedure.

Specifically, 1 µg in terms of protein of the anti-SARS-CoV-2 antibody (hereinafter, the weight in terms of protein is represented by a weight value obtained by gravimetric analysis of the purified protein) and 1 mL of the platinum-gold colloid suspension liquid prepared in (1) were mixed, and the mixture was placed still at room temperature for 4 minutes, causing the anti-SARS-CoV-2 antibody to bind to the surfaces of the platinum-gold colloid particles. Thereafter, a 5% aqueous BSA solution was added in an amount corresponding to a final concentration of 0.5% with respect to the total liquid amount, and free surface regions of the platinum-gold colloid particles were blocked with BSA to prepare a platinum-gold colloid-labeled anti-SARS-CoV-2 antibody solution. Thereafter, the solution was centrifuged (5,600 × g, 25 minutes) to precipitate the platinum-gold colloid-labeled antibody, and the supernatant was removed to acquire a platinum-gold colloid-labeled antibody. The obtained platinum-gold colloid-labeled antibody was suspended in a 50 mM tris-hydrochloric acid buffer solution (pH 7.4) containing 10% sucrose, 1% BSA and 0.5% Triton-X100, thereby obtaining a platinum-gold colloid-labeled antibody solution.

### (3) Preparation of immunochromatographic test strip for detection of SARS-CoV-2

For preparing an immunochromatographic test strip, a nitrocellulose membrane as a chromatographic development membrane carrier, glass fiber nonwoven fabric as an impregnation member for a platinum-gold colloid-labeled antibody, cotton cloth as a member for adding sample, and filter paper as an absorbing member were provided. A solution containing an anti-SARS-CoV-2 antibody at 1.0 mg/mL was linearly applied in an amount of 0.5 µL to the chromatographic development membrane carrier at a position of 7.5 mm from its end on the chromatographic development start point side with an antibody application apparatus (Musashi engineering Co., Ltd.), and dried at room temperature to prepare an immunochromatography membrane.

Next, the glass fiber nonwoven fabric was impregnated with the platinum-gold colloid-labeled antibody solution in an amount of 37.5 µL, and freeze-dried to prepare a platinum-gold colloid-labeled antibody impregnation member.

An immunochromatographic test strip similar to that of Figure 1 was prepared using the chromatographic development membrane carrier and labeled antibody impregnation member prepared as described above, and the member for adding sample and the absorbing member.

### Example 12: Test for reactivity to recombinant SARS-CoV-2 nucleocapsid protein using immunochromatographic test strip

According to the method described in Example 11, immunochromatographic test strips were prepared in which the SARS-CoV-2 antibodies obtained in Example 3 were used in various combinations as the first antibody (immobilized antibody) that was immobilized on the membrane carrier and the second antibody (labeled antibody) that was a platinum-gold colloid-labeled antibody. Using the immunochromatographic test strips, reactivity tests were conducted. Evaluation of the test strip was performed based on whether the determination part had a color after 15 minutes or not. The color results were given according to the four levels of: no color (-); faint color (±); sufficiently dense color (+); and significantly dense color (++). In general, when the faint color is obtained, the color is at a degree that can be visually confirmed.

### (1) Test for reactivity of immunochromatographic test strip using TAU002 as the second antibody (labeled antibody), and TAU001 or TAU004 as the first antibody (immobilized antibody)

According to the method described in Example 11, the anti-SARS-CoV-2 antibody TAU002 was labeled with platinum-gold colloid, and TAU001 or TAU004 was immobilized on the membrane carrier. With these antibodies, immunochromatographic test strips were prepared, and to the prepared test strips, 90 µL of SARS-CoV-2 recombinant NP at 4 ng/mL was added dropwise as an antigen to evaluate the reactivity. Table 8 shows the results.

**Table 8**

| Evaluation of reactivity using TAU002 as labeled antibody | | | |
|---|---|---|---|
| Labeled antibody | Immobilized antibody | Blank | With antigen |
| TAU002 | TAU001 | - | + |
| | TAU004 | - | + |

The results of Table 8 showed that when TAU002 was used as the platinum-gold colloid labeled antibody, color was shown on the membrane carrier on which TAU001 or TAU004 was immobilized, and therefore the nucleocapsid protein of SARS-CoV-2 could be detected. Further, it was confirmed that the reactivity slightly varied depending on a combination of the labeled antibody and the immobilized antibody.

### (2) Test for reactivity of immunochromatographic test strip using TAU003 as the second antibody (labeled antibody) and TAU001 or TAU004 as the first antibody (immobilized antibody)

According to the method described in Example 11, the anti-SARS-CoV-2 antibody TAU003 was labeled with platinum-gold colloid, and TAU001 or TAU004 was immobilized on the membrane carrier. With these antibodies, immunochromatographic test strips were prepared, and to the prepared test strips, 90 µL of SARS-CoV-2 recombinant NP at 4 ng/mL was added dropwise as an antigen to evaluate the reactivity. Table 9 shows the results.

**Table 9**

| Evaluation of reactivity using TAU003 as labeled antibody | | | |
|---|---|---|---|
| Labeled antibody | Immobilized antibody | Blank | With antigen |
| TAU003 | TAU001 | - | + |
| | TAU004 | - | ++ |

The results of Table 9 showed that when TAU003 was used as the platinum-gold colloid labeled antibody, color was shown on the membrane carrier on which TAU001 or TAU004 was immobilized, and therefore the nucleocapsid protein of SARS-CoV-2 could be detected. Comparison with Table 8 resulting from a similarly conducted test showed that the detection sensitivity could be improved by using TAU003 as the labeled antibody.

### (3) Test for reactivity of immunochromatographic test strip using TAU004 as the second antibody (labeled antibody) and TAU001, TAU002, TAU003, TAU005 or TAU006 as the first antibody (immobilized antibody)

According to the method described in Example 11, the anti-SARS-CoV-2 antibody TAU004 was labeled with platinum-gold colloid, and TAU001, TAU002, TAU003, TAU005 or TAU006 was immobilized on the membrane carrier. With these antibodies, immunochromatographic test strips were prepared, and to the prepared test strips, 90 µL of SARS-CoV-2 recombinant NP at 4 ng/mL was added dropwise as an antigen to evaluate the reactivity. Table 10 shows the results.

**Table 10**

| Evaluation of reactivity using TAU004 as labeled antibody | | | |
|---|---|---|---|
| Labeled antibody | Immobilized antibody | Blank | With antigen |
| TAU004 | TAU001 | - | + |
| | TAU002 | - | + |
| | TAU003 | - | ++ |
| | TAU005 | - | ++ |
| | TAU006 | - | ++ |

The results of Table 10 showed that when TAU004 was used as the platinum-gold colloid-labeled antibody, color was shown on the membrane carrier on which any of the anti-SARS-CoV-2 antibodies was immobilized, and therefore the nucleocapsid protein of SARS-CoV-2 could be detected in any of the combinations. In addition, it was shown that when TAU004 was used as the platinum-gold colloid-labeled antibody, TAU004 could be used in combination with various antibodies. Comparison with Table 9 resulting from a similarly conducted test showed that when TAU004 was used as the labeled antibody, TAU004 could be combined with various antibodies while improving the detection sensitivity.

### (4) Test for reactivity of immunochromatographic test strip using TAU005 as the second antibody (labeled antibody) and TAU001 or TAU004 as the first antibody (immobilized antibody)

According to the method described in Example 11, the anti-SARS-CoV-2 antibody TAU005 was labeled with platinum-gold colloid, and TAU001 or TAU004 was immobilized on the membrane carrier. With these antibodies, immunochromatographic test strips were prepared, and to the prepared test strips, 90 µL of SARS-CoV-2 recombinant NP at 4 ng/mL was added dropwise as an antigen to evaluate the reactivity. Table 11 shows the results.

**Table 11**

| Evaluation of reactivity using TAU005 as labeled antibody | | | |
|---|---|---|---|
| Labeled antibody | Immobilized antibody | Blank | With antigen |
| TAU005 | TAU001 | - | + |
| | TAU004 | - | ++ |

The results of Table 11 showed that when TAU005 was used as the platinum-gold colloid labeled antibody, color was shown on the membrane carrier on which TAU001 and TAU004 was immobilized, and therefore the nucleocapsid protein of SARS-CoV-2 could be detected. However, the results were equivalent to those shown in Table 9 in which TAU003 was used.

### (5) Test for reactivity of immunochromatographic test strip using TAU006 as the second antibody (labeled antibody) and TAU001 or TAU004 as the first antibody (immobilized antibody)

According to the method described in Example 11, the anti-SARS-CoV-2 antibody TAU005 was labeled with platinum-gold colloid, and TAU001 or TAU004 was immobilized on the membrane carrier. With these antibodies, immunochromatographic test strips were prepared, and to the prepared test strips, 90 µL of SARS-CoV-2 recombinant NP at 4 ng/mL was added dropwise as an antigen to evaluate the reactivity. Table 12 shows the results.

**Table 12**

| Evaluation of reactivity using TAU006 as labeled antibody | | | |
|---|---|---|---|
| Labeled antibody | Immobilized antibody | Blank | With antigen |
| TAU006 | TAU001 | - | + |
| | TAU004 | - | + |

The results of Table 12 showed that when TAU006 was used as the platinum-gold colloid labeled antibody, color was shown on the membrane carrier on which TAU001 or TAU004 was immobilized, and therefore the nucleocapsid protein of SARS-CoV-2 could be detected. Use of TAU006 provided results equivalent to those obtained above by using the TAU002 as the labelled antibody.

The results obtained in Example 12 showed that when two types of antibodies were selected from the anti-SARS-CoV-2 antibodies obtained in Example 3 and used to prepare an immunochromatographic test strip, color was observed in various combinations of antibodies. In addition, it was shown that the combination of antibodies enables more sensitive detection of the nucleocapsid protein of SARS-CoV-2. Further, it was shown that when TAU004 was used either as the first antibody which was an immobilized antibody or the second antibody which was a labeled antibody, TAU004 could be combined with various antibodies while SARS-CoV-2 could be detected with high sensitivity.

### Example 13: Evaluation of reactivity of immunochromatographic test strip using specimen

According to the method described in Example 11, the anti-SARS-CoV-2 antibody prepared in Example 10 was labeled with platinum-gold colloid, and a different anti-SARS-CoV-2 antibody was immobilized on the membrane carrier to prepare an immunochromatographic test strip. To the prepared test strip, 90 µL of a positive specimen was added dropwise as a biological sample to evaluate the reactivity. Here, as specimen types, a nasopharynx swab specimen and a saliva specimen were used. Evaluation of the test strip was performed based on whether the determination part had a color after 15 minutes or not. The color results were determined according to the four levels of: no color (-); faint color (±); sufficiently dense color (+); and significantly dense color (++). Tables 13 and 14 show the results.

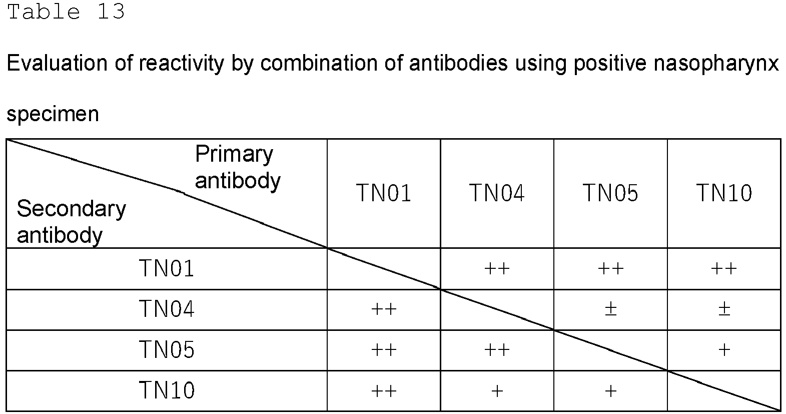

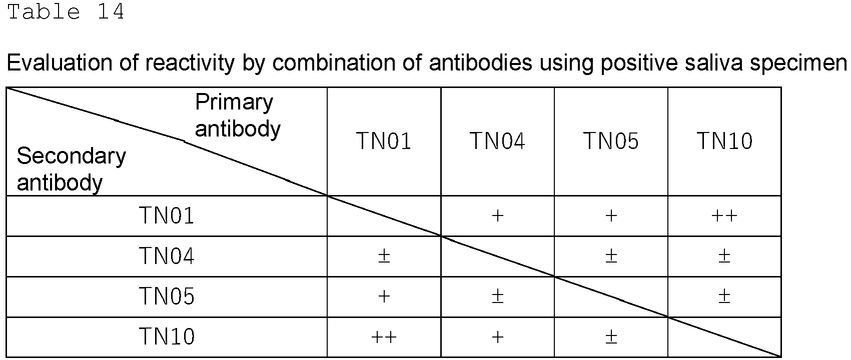

As shown in Tables 13 and 14, color was observed in many combinations regardless of which specimen was used. It is generally said that detection is more difficult when a saliva specimen is used than when a nasopharynx specimen is used. However, even when a saliva specimen was used, the SARS-CoV-2 nucleocapsid protein contained in the specimen was detected by combining the antibodies of the present invention. This showed that even when a saliva specimen was used, an immunochromatographic test strip using the anti-SARS-CoV-2 antibody obtained in Example 10 exhibited SARS-CoV-2 nucleocapsid protein-detecting performance as in the case of using a nasopharynx specimen.

According to the same method as described above, the performance of the immunochromatographic test strip was evaluated by performing immunochromatography using the SARS-CoV-2 antibodies prepared in Examples 3 and 10 in various combinations. Evaluation of the test strip was performed based on whether the determination part had a color after 15 minutes or not. The color results were determined according to the four levels of: no color (-); faint color (±); sufficiently dense color (+); and significantly dense color (++). Tables 15 to 23 show the results. Here, as the specimen, a SARS-CoV-2-positive nasopharynx specimen was used.

**Table 15**

| Evaluation of reactivity of immunochromatographic test strip using TAU007 as the second antibody (labeled antibody) | | | |
|---|---|---|---|
| Labeled antibody | Immobilized antibody | Blank | With antigen |
| TAU007 | TAU008 | - | ++ |
| | TN03 | - | ++ |
| | TN05 | - | + |
| | TN07 | - | ++ |
| | TN08 | - | ++ |
| | TN09 | - | + |

**Table 16**

| Evaluation of reactivity of immunochromatographic test strip using TAU008 as the second antibody (labeled antibody) | | | |
|---|---|---|---|
| Labeled antibody | Immobilized antibody | Blank | With antigen |
| TAU008 | TAU007 | - | ++ |
| | TN01 | - | ++ |
| | TN03 | - | + |
| | TN04 | - | ++ |
| | TN05 | - | ++ |
| | TN07 | - | + |
| | TN08 | - | ++ |
| | TN09 | - | ++ |
| | TN10 | - | ++ |

**Table 17**

| Evaluation of reactivity of immunochromatographic test strip using TN01 as the second antibody (labeled antibody) | | | |
|---|---|---|---|
| Labeled antibody | Immobilized antibody | Blank | With antigen |
| TN01 | TAU007 | - | ++ |
| | TAU008 | - | ++ |
| | TN04 | - | ++ |
| | TN05 | - | ++ |
| | TN09 | - | ++ |
| | TN10 | - | ++ |

**Table 18**

| Evaluation of reactivity of immunochromatographic test strip using TN03 as the second antibody (labeled antibody) | | | |
|---|---|---|---|
| Labeled antibody | Immobilized antibody | Blank | With antigen |
| TN03 | TAU007 | - | + |
| | TAU008 | - | ++ |
| | TN04 | - | ++ |
| | TN05 | - | ++ |
| | TN09 | - | ++ |
| | TN10 | - | ++ |

**Table 19**

| Evaluation of reactivity of immunochromatographic test strip using TN04 as the second antibody (labeled antibody) | | | |
|---|---|---|---|
| Labeled antibody | Immobilized antibody | Blank | With antigen |
| TN04 | TAU007 | - | + |
| | TAU008 | - | ++ |
| | TN01 | - | ++ |
| | TN03 | - | ++ |
| | TN05 | - | + |
| | TN07 | - | + |
| | TN08 | - | + |
| | TN09 | - | + |
| | TN10 | - | + |

**Table 20**

| Evaluation of reactivity of immunochromatographic test strip using TN05 as the second antibody (labeled antibody) | | | |
|---|---|---|---|
| Labeled antibody | Immobilized antibody | Blank | With antigen |
| TN05 | TAU007 | - | + |
| | TAU008 | - | + |
| | TN01 | - | ++ |
| | TN03 | - | + |
| | TN04 | - | ++ |
| | TN07 | - | + |
| | TN08 | - | + |
| | TN10 | - | + |

**Table 21**

| Evaluation of reactivity of immunochromatographic test strip using TN08 as the second antibody (labeled antibody) | | | |
|---|---|---|---|
| Labeled antibody | Immobilized antibody | Blank | With antigen |
| TN08 | TAU007 | | ++ |
| | TAU008 | | ++ |
| | TN04 | | ++ |
| | TN05 | | + |
| | TN09 | | ++ |

**Table 22**

| Evaluation of reactivity of immunochromatographic test strip using TN09 as the second antibody (labeled antibody) | | | |
|---|---|---|---|
| Labeled antibody | Immobilized antibody | Blank | With antigen |
| TN09 | TAU007 | - | + |
| | TAU008 | - | ++ |
| | TN01 | - | ++ |
| | TN03 | - | ++ |
| | TN04 | - | ++ |
| | TN07 | - | ++ |
| | TN08 | - | ++ |
| | TN10 | - | + |

**Table 23**

| Evaluation of reactivity of immunochromatographic test strip using TN10 as the second antibody (labeled antibody) | | | |
|---|---|---|---|
| Labeled antibody | Immobilized antibody | Blank | With antigen |
| TN10 | TAU007 | - | + |
| | TAU008 | - | ++ |
| | TN01 | - | ++ |
| | TN03 | - | ++ |
| | TN04 | - | + |
| | TN05 | - | + |
| | TN07 | - | + |
| | TN09 | - | + |

The results of Tables 15 to 23 show that color was observed in various combinations of antibodies regardless of which antibody was used as the second antibody. When TAU007 is used as the second antibody, the first antibody is preferably TAU008, TN03, TN05, TN07, TN08, TN09.

When TAU008 is used as the second antibody, the first antibody is preferably TAU007, TN01, TN03, TN04, TN05, TN07, TN08, TN09, TN10.

When TN01 is used as the second antibody, the first antibody is preferably TAU007, TAU008, TN04, TN05, TN09, TN10.

When TN03 is used as the second antibody, the first antibody is preferably TAU007, TAU008, TN04, TN05, TN09, TN10.

When TN04 is used as the second antibody, the first antibody is preferably TAU007, TAU008, TN01, TN03, TN05, TN07, TN08, TN09, TN10.

When TN05 is used as the second antibody, the first antibody is preferably TAU007, TAU008, TN01, TN03, TN04, TN07, TN08, TN10.

When TN08 is used as the second antibody, the first antibody is preferably TAU007, TAU008, TN04, TN05, TN09.

When TN09 is used as the second antibody, the first antibody is preferably TAU007, TAU008, TN01, TN03, TN04, TN07, TN08, TN10.

When TN10 is used as the second antibody, the first antibody is preferably TAU007, TAU008, TN01, TN03, TN04, TN05, TN07, TN09.

From the above, it was shown that antigens contained in the nasopharynx specimen could be detected by various combinations of the obtained antibodies.

Further, for the SARS-CoV-2-positive saliva specimen, the SARS-CoV-2 antibodies prepared in Examples 3 and 8 were used in various combinations, and measurement was performed by an immunochromatography method to evaluate the performance of the immunochromatographic test strip on the saliva specimen. Evaluation of the test strip was performed based on whether the determination part had a color after 15 minutes or not. The color results were determined according to the four levels of: no color (-); faint color (±); sufficiently dense color (+); and significantly dense color (++). Tables 24 to 32 show the results.

**Table 24**

| Evaluation of reactivity of immunochromatographic test strip using TAU007 as the second antibody (labeled antibody) | | | |
|---|---|---|---|
| Labeled antibody | Immobilized antibody | Blank | With antigen |
| TAU007 | TAU008 | - | ++ |
| | TN03 | - | ++ |
| | TN05 | - | + |
| | TN07 | - | + |
| | TN08 | - | ++ |
| | TN09 | - | + |

**Table 25**

| Evaluation of reactivity of immunochromatographic test strip using TAU008 as the second antibody (labeled antibody) | | | |
|---|---|---|---|
| Labeled antibody | Immobilized antibody | Blank | With antigen |
| TAU008 | TAU007 | - | ++ |
| | TN01 | - | ++ |
| | TN03 | - | ++ |
| | TN04 | - | ++ |
| | TN05 | - | + |
| | TN07 | - | ++ |
| | TN08 | - | ++ |
| | TN09 | - | ++ |
| | TN10 | - | ++ |

**Table 26**

| Evaluation of reactivity of immunochromatographic test strip using TN01 as the second antibody (labeled antibody) | | | |
|---|---|---|---|
| Labeled antibody | Immobilized antibody | Blank | With antigen |
| TN01 | TN04 | - | + |
| | TN05 | - | + |
| | TN09 | - | + |
| | TN10 | - | ++ |

**Table 27**

| Evaluation of reactivity of immunochromatographic test strip using TN03 as the second antibody (labeled antibody) | | | |
|---|---|---|---|
| Labeled antibody | Immobilized antibody | Blank | With antigen |
| TN03 | TAU007 | - | + |
| | TAU008 | - | ++ |
| | TN04 | - | ++ |
| | TN05 | - | + |
| | TN09 | - | ++ |
| | TN10 | - | ++ |

**Table 28**

| Evaluation of reactivity of immunochromatographic test strip using TN04 as the second antibody (labeled antibody) | | | |
|---|---|---|---|
| Labeled antibody | Immobilized antibody | Blank | With antigen |
| TN04 | TAU007 | - | + |
| | TAU008 | - | + |
| | TN01 | - | + |
| | TN03 | - | + |
| | TN05 | - | + |
| | TN07 | - | ± |
| | TN08 | - | + |
| | TN09 | - | + |
| | TN10 | - | + |

**Table 29**

| Evaluation of reactivity of immunochromatographic test strip using TN05 as the second antibody (labeled antibody) | | | |
|---|---|---|---|
| Labeled antibody | Immobilized antibody | Blank | With antigen |
| TN05 | TAU008 | - | + |
| | TN01 | - | + |
| | TN03 | - | + |
| | TN04 | - | + |
| | TN07 | - | + |
| | TN08 | - | ± |
| | TN10 | - | + |

**Table 30**

| Evaluation of reactivity of immunochromatographic test strip using TN08 as the second antibody (labeled antibody) | | | |
|---|---|---|---|
| Labeled antibody | Immobilized antibody | Blank | With antigen |
| TN08 | TAU007 | - | ++ |
| | TAU008 | - | ++ |
| | TN04 | - | ++ |
| | TN05 | - | + |
| | TN09 | - | + |

**Table 31**

| Evaluation of reactivity of immunochromatographic test strip using TN09 as the second antibody (labeled antibody) | | | |
|---|---|---|---|
| Labeled antibody | Immobilized antibody | Blank | With antigen |
| TN09 | TAU007 | - | + |
| | TAU008 | - | + |
| | TN01 | - | + |
| | TN03 | - | + |
| | TN04 | - | + |
| | TN07 | - | + |
| | TN08 | - | + |
| | TN10 | - | + |

**Table 32**

| Evaluation of reactivity of immunochromatographic test strip using TN10 as the second antibody (labeled antibody) | | | |
|---|---|---|---|
| Labeled antibody | Immobilized antibody | Blank | With antigen |
| TN10 | TAU007 | - | + |
| | TAU008 | - | ++ |
| | TN01 | - | ++ |
| | TN03 | - | ++ |
| | TN04 | - | + |
| | TN05 | - | + |
| | TN07 | - | + |
| | TN09 | - | + |

The results of Tables 24 to 32 show that even when the saliva specimen was used, color was observed in various combinations of antibodies regardless of which antibody was used as the second antibody. When TAU007 is used as the second antibody, the first antibody is preferably TAU008, TN03, TN05, TN07, TN08, TN09.

When TAU008 is used as the second antibody, the first antibody is preferably TAU007, TN01, TN03, TN04, TN05, TN07, TN08, TN09, TN10.

When TN01 is used as the second antibody, the first antibody is preferably TN04, TN05, TN09, TN10.

When TN03 is used as the second antibody, the first antibody is preferably TAU007, TAU008, TN04, TN05, TN09, TN10.

When TN04 is used as the second antibody, the first antibody is preferably TAU007, TAU008, TN01, TN03, TN05, TN07, TN08, TN09, TN10.

When TN05 is used as the second antibody, the first antibody is preferably TAU008, TN01, TN03, TN04, TN07, TN08, TN10.

When TN08 is used as the second antibody, the first antibody is preferably TAU007, TAU008, TN04, TN05, TN09.

When TN09 is used as the second antibody, the first antibody is preferably TAU007, TAU008, TN01, TN03, TN04, TN07, TN08, TN10.

When TN10 is used as the second antibody, the first antibody is preferably TAU007, TAU008, TN01, TN03, TN04, TN05, TN07, TN09.

From the above, it was shown that antigens contained in the saliva specimen could be detected by various combinations of the obtained antibodies. In addition, it was shown that there are combinations of antibodies in which color density differed between cases where the nasopharynx specimen was used and where the saliva specimen was used. However, regardless of the specimen type, all the listed combinations of antibodies enabled detection of the SARS-CoV-2 nucleocapsid protein contained in the specimen. Thus, by combining the antibodies of the present invention, the SARS-CoV-2 nucleocapsid protein contained in the nasopharynx specimen and the saliva specimen can be detected with high sensitivity.

### Example 14: Evaluation of nonspecific reactivity of immunochromatographic test strip using negative specimen

According to the method described in Example 11, the anti-SARS-CoV-2 antibody TAU004 was taken as the second antibody and labeled with platinum-gold colloid, and TAU005 was taken as the first antibody and immobilized on the membrane carrier to prepare an immunochromatographic test strip. To the prepared test strip, 90 µL of a negative specimen as a biological sample was added dropwise to evaluate the reactivity. Here, as specimen types, a nasopharynx specimen and a saliva specimen were used, and 50 specimens were tested for each specimen type. Evaluation of the test strip was performed based on whether the determination part had a color after 15 minutes or not. The color results were given according to the four levels of: no color (-); faint color (±); sufficiently dense color (+); and significantly dense color (++). When a faint color was obtained, it was determined that a nonspecific reaction occurred. Table 33 shows the results.

**Table 33**

| Evaluation of nonspecific reaction in nasopharynx specimen and saliva specimen | | |
|---|---|---|
| Specimen No. | Nasopharynx swab specimen | Saliva specimen |
| 1 | - | - |
| 2 | - | - |
| 3 | - | - |
| 4 | - | - |
| 5 | - | - |
| 6 | - | - |
| 7 | - | - |
| 8 | - | - |
| 9 | - | - |
| 10 | - | - |
| 11 | - | - |
| 12 | - | - |
| 13 | - | - |
| 14 | - | - |
| 15 | - | - |
| 16 | - | - |
| 17 | - | - |
| 18 | - | - |
| 19 | - | - |
| 20 | - | - |
| 21 | - | - |
| 22 | - | - |
| 23 | - | - |
| 24 | - | - |
| 25 | - | - |
| 26 | - | - |
| 27 | - | - |
| 28 | - | - |
| 29 | - | - |
| 30 | - | - |
| 31 | - | - |
| 32 | - | - |
| 33 | - | - |
| 34 | - | - |
| 35 | - | - |
| 36 | - | - |
| 37 | - | - |
| 38 | - | - |
| 39 | - | - |
| 40 | - | - |
| 41 | - | - |
| 42 | - | - |
| 43 | - | - |
| 44 | - | - |
| 45 | - | - |
| 46 | - | - |
| 47 | - | - |
| 48 | - | - |
| 49 | - | - |
| 50 | - | - |

The results of Table 33 showed that when negative specimens of various specimen types were added dropwise to the prepared immunochromatographic test strip, color was not observed in any of the 50 specimens. Thus, regardless of the specimen type, a nonspecific reaction by a cross reaction with specimen components was not observed in the immunochromatographic test strip using TAU004 and TAU005.

For these specimens, a nucleic acid amplification method (PCR method) based on a gene test method developed by National Institute of Infectious Diseases (SARS-CoV-2 Gene Detection/Virus Separation Manual Ver. 1.1) was carried out, and as a result, it was confirmed that all the specimens were negative.

### Example 15: Evaluation of reactivity of immunochromatographic test strip using clinical sample

The nasopharynx of a patient clinically suspected of infection with SARS-CoV-2 was scraped with a sterile cotton swab to collect a nasopharynx swab. The cotton swab used for collection of the nasopharynx swab was subjected to extraction with an analyte extracting liquid to prepare a test sample. Detection of the nucleocapsid protein of SARS-CoV-2 in the test sample was made by using an immunochromatographic test strip prepared in the same manner as in Example 14. As a control, ImmunoAce (trademark) SARS-CoV-2 (antigen qualitative detection kit, TAUNS Laboratories, Inc.) sold as a product using antibodies different from the antibodies of the present invention was used. In addition, the collected clinical samples were subjected to a nucleic acid amplification method (PCR method) based on a gene test method developed by National Institute of Infectious Diseases (SARS-CoV-2 Gene Detection/Virus Separation Manual Ver. 1.1) to examine the presence of SARS-CoV-2. Table 34 shows the results of visual determination in immunochromatography using the test samples, and the Ct values of the test samples in the PCR method. Evaluation of the test strip was performed based on whether the determination part had a color after 15 minutes or not. The color results were given according to the four levels of: no color (-); faint color (±); sufficiently dense color (+); and significantly dense color (++). "ND" was written for the PCR result without detection, and a Ct value was written for the PCR result with detection.

**Table 34**

| | ImmunoAce SARS-CoV-2 | Test strip prepared in Example 14 | PCR Ct value |
|---|---|---|---|
| Clinical sample 1 | - | - | ND |
| Clinical sample 2 | ++ | ++ | 15.60 |
| Clinical sample 3 | + | ++ | 25.98 |
| Clinical sample 4 | ++ | ++ | 23.06 |
| Clinical sample 5 | - | - | 42.91 |
| Clinical sample 6 | - | - | 39.26 |
| Clinical sample 7 | - | - | 34.29 |
| Clinical sample 8 | - | + | 33.63 |
| Clinical sample 9 | - | - | ND |
| Clinical sample 10 | - | ++ | 24.08 |

The results of Table 34 showed that in ImmunoAce (trademark) SARS-CoV-2, color was observed for three of ten samples. On the other hand, when the same samples were reacted using the test plate prepared in Example 14, color was observed for five samples. The Ct values of two samples for which ImmunoAce (trademark) SARS-CoV-2 were unable to detect were 33.63 and 24.08, respectively. This showed that the test plate prepared in Example 14 had higher detection sensitivity over the antigen detection kit previously sold as a product. The improvement of detection sensitivity was also demonstrated by the fact that the color strength for the clinical sample 3 increased. For clinical samples 1 and 9 without PCR detection, color was not observed regardless of which test plate was used, and therefore the test strip was thought to have low cross reactivity with other components contained in the sample.

### Example 16: Evaluation of reactivity of immunochromatographic test strip using saliva sample

According to the method described in Example 12, the anti-SARS-CoV-2 antibody TAU008 was taken as the second antibody and labeled with platinum-gold colloid, and TAU007 was taken as the first antibody and immobilized on the membrane carrier to prepare an immunochromatographic test strip. Saliva spat from a patient clinically suspected of infection with SARS-CoV-2 was collected by absorption into a sterile cotton swab, and the cotton swab used for collection of the saliva was subjected to extraction with an analyte extracting liquid to obtain a test sample for the prepared immunochromatographic test strip. In parallel, the nasopharynx of the same patient was scraped with a sterile cotton swab to collect a nasopharynx swab. The cotton swab used for collection of the nasopharynx swab was subjected to extraction with an analyte extracting liquid to obtain a test sample for ImmunoAce (trademark) SARS-CoV-2 (TAUNS Laboratories, Inc.). Performance of ImmunoAce (trademark) SARS-CoV-2 (TAUNS Laboratories, Inc.) and the prepared immunochromatographic test strip for the nucleocapsid protein of SARS-CoV-2 contained in test samples was evaluated using the respective test samples that were prepared as above. Here, ImmunoAce (trademark) SARS-CoV-2 (TAUNS Laboratories, Inc.) was used as a control. In addition, a nucleic acid amplification method (PCR method) based on a gene test method developed by National Institute of Infectious Diseases (SARS-CoV-2 Gene Detection/Virus Separation Manual Ver. 1.1) was carried out for the collected clinical samples to examine the presence of SARS-CoV-2. Table 35 shows the results of visual determination in immunochromatography using the test samples, and the Ct values of the test samples in the PCR method. Evaluation of the test strip was performed based on whether the determination part had a color after 15 minutes or not. The color results were given according to the four levels of: no color (-); faint color (±); sufficiently dense color (+); and significantly dense color (++). "ND" was written for the PCR result without detection, and a Ct value was written for the PCR result with detection.

**Table 35**

| Comparison between nasopharynx sample and saliva sample collected from same patient | | | | |
|---|---|---|---|---|
| | Nasopharynx sample | | Saliva sample | |
| | ImmunoAce SARS-CoV-2 | PCR Ct value | Test strip prepared in Example 16 | PCR Ct value |
| Subject 1 | - | ND | - | ND |
| Subject 2 | ++ | 27.50 | ++ | 22.45 |
| Subject 3 | + | 36.29 | ++ | 32.29 |
| Subject 4 | + | 28.13 | + | 27.21 |
| Subject 5 | - | 37.70 | - | ND |
| Subject 6 | ++ | 20.09 | ++ | 17.90 |
| Subject 7 | - | ND | - | 37.45 |
| Subject 8 | - | 32.16 | - | 33.93 |
| Subject 9 | + | 33.95 | ++ | 31.70 |
| Subject 10 | - | 24.85 | - | 33.07 |
| Subject 11 | ++ | 31.67 | ++ | 27.00 |
| Subject 12 | ++ | 20.78 | ++ | 25.60 |
| Subject 13 | - | 27.10 | - | 30.08 |
| Subject 14 | - | 35.01 | + | 28.56 |
| Subject 15 | - | 32.82 | + | 25.96 |
| Subject 16 | + | 31.34 | + | 29.17 |
| Subject 17 | + | 38.20 | + | 31.99 |
| Subject 18 | - | ND | - | ND |
| Subject 19 | ++ | 32.92 | ++ | 25.42 |
| Subject 20 | - | ND | - | ND |
| Subject 21 | + | 26.25 | + | 29.66 |
| Subject 22 | ++ | 29.34 | ++ | 27.31 |
| Subject 23 | ++ | 27.49 | ++ | 23.11 |
| Subject 24 | ++ | 18.68 | ++ | 20.46 |
| Subject 25 | - | 17.53 | + | 24.07 |
| Subject 26 | ++ | 27.88 | ++ | 27.65 |
| Subject 27 | + | 31.93 | + | 28.68 |
| Subject 28 | ++ | 29.62 | ++ | 19.68 |
| Subject 29 | - | ND | - | ND |
| Subject 30 | + | 29.60 | ++ | 32.16 |

From Table 35, it was shown that for ImmunoAce (trademark) SARS-CoV-2 with the nasopharynx sample, color was observed in eighteen of thirty samples. On the other hand, when the saliva sample from the same subject was reacted using the test strip prepared in Example 16, color was observed in twenty one of thirty samples. Thus, even when the saliva sample was used, it was possible to perform detection as in the case of use of the nasopharynx sample. For subjects 25 and 30 in which the amount of virus contained in the nasopharynx was large and the amount of virus contained in the saliva was small, the test strip prepared in the present Example gave a strong color. From the above, the test strip prepared in the present Example was shown to have higher detection sensitivity over the antigen detection kit previously sold as a product. For samples having no PCR detection, color was not observed when the immunochromatographic test strip prepared in the present Example was used, and therefore it was thought that it was possible to suppress nonspecific color while improving the detection sensitivity.

### Industrial Applicability

The present invention provides an antibody against the nucleocapsid protein of SARS-CoV-2, a method for immunologically detecting SARS-CoV-2 using the antibody, and a kit using the antibody. This enables rapid and specific detection of infection with SARS-CoV-2 without requiring special equipment or any high level of technical skill.

### Reference Signs List

1 adhesive sheet
2 impregnation member
3 membrane carrier
31 capture portion
32 control capture portion
4 absorbing member
5 member for adding sample

## Claims

1. A method for detecting a presence of SARS-CoV-2 contained in a test sample, which comprises performing an immunoassay using an antibody or a fragment thereof that reacts with a nucleocapsid protein of the SARS-CoV-2.

2. The method for detecting SARS-CoV-2 according to claim 1, wherein the immunoassay comprises contacting the test sample with the antibody or the fragment thereof.

3. The method for detecting SARS-CoV-2 according to claim 2, wherein the immunoassay is a sandwich method using the two different antibodies or fragments thereof.

4. The method for detecting SARS-CoV-2 according to claim 3, wherein the sandwich method is an immunochromatography method or an ELISA method.

5. The method for detecting SARS-CoV-2 according to any one of claims 1 to 4, wherein the antibody or fragment thereof is selected from the antibodies or fragments thereof described in (1) to (20) below:
(1) an antibody in which CDR1, CDR2 and CDR3 of a heavy chain comprise amino acid sequences set forth as SEQ ID NOS: 2, 3 and 4, respectively, and CDR1, CDR2 and CDR3 of a light chain comprise amino acid sequences set forth as SEQ ID NOS: 5, 6 and 7, respectively, or a fragment thereof;
(2) an antibody in which CDR1, CDR2 and CDR3 of a heavy chain comprise amino acid sequences set forth as SEQ ID NOS: 8, 9 and 10, respectively, and CDR1, CDR2 and CDR3 of a light chain comprise amino acid sequences set forth as SEQ ID NOS: 11, 12 and 13, respectively, or a fragment thereof;
(3) an antibody in which CDR1, CDR2 and CDR3 of a heavy chain comprise amino acid sequences set forth as SEQ ID NOS: 14, 15 and 16, respectively, and CDR1, CDR2 and CDR3 of a light chain comprise amino acid sequences set forth as SEQ ID NOS: 17, 18 and 19, respectively, or a fragment thereof;
(4) an antibody in which CDR1, CDR2 and CDR3 of a heavy chain comprise amino acid sequences set forth as SEQ ID NOS: 20, 21 and 22, respectively, and CDR1, CDR2 and CDR3 of a light chain comprise amino acid sequences set forth as SEQ ID NOS: 23, 24 and 25, respectively, or a fragment thereof;
(5) an antibody in which CDR1, CDR2 and CDR3 of a heavy chain comprise amino acid sequences set forth as SEQ ID NOS: 26, 27 and 28, respectively, and CDR1, CDR2 and CDR3 of a light chain comprise amino acid sequences set forth as SEQ ID NOS: 29, 30 and 31, respectively, or a fragment thereof;
(6) an antibody in which CDR1, CDR2 and CDR3 of a heavy chain comprise amino acid sequences set forth as SEQ ID NOS: 32, 33 and 34, respectively, and CDR1, CDR2 and CDR3 of a light chain comprise amino acid sequences set forth as SEQ ID NOS: 35, 36 and 37, respectively, or a fragment thereof;
(7) an antibody in which CDR1, CDR2 and CDR3 of a heavy chain comprise amino acid sequences set forth as SEQ ID NOS: 38, 39 and 40, respectively, and CDR1, CDR2 and CDR3 of a light chain comprise amino acid sequences set forth as SEQ ID NOS: 41, 42 and 43, respectively, or a fragment thereof;
(8) an antibody in which CDR1, CDR2 and CDR3 of a heavy chain comprise amino acid sequences set forth as SEQ ID NOS: 44, 45 and 46, respectively, and CDR1, CDR2 and CDR3 of a light chain comprise amino acid sequences set forth as SEQ ID NOS: 47, 48 and 49, respectively, or a fragment thereof;
(9) an antibody in which CDR1, CDR2 and CDR3 of a heavy chain comprise amino acid sequences set forth as SEQ ID NOS: 50, 51 and 52, respectively, and CDR1, CDR2 and CDR3 of a light chain comprise amino acid sequences set forth as SEQ ID NOS: 53, 54 and 55, respectively, or a fragment thereof;
(10) an antibody in which CDR1, CDR2 and CDR3 of a heavy chain comprise amino acid sequences set forth as SEQ ID NOS: 56, 57 and 58, respectively, and CDR1, CDR2 and CDR3 of a light chain comprise amino acid sequences set forth as SEQ ID NOS: 59, 60 and 61, respectively, or a fragment thereof;
(11) an antibody in which CDR1, CDR2 and CDR3 of a heavy chain comprise amino acid sequences set forth as SEQ ID NOS: 62, 63 and 64, respectively, and CDR1, CDR2 and CDR3 of a light chain comprise amino acid sequences set forth as SEQ ID NOS: 65, 66 and 67, respectively, or a fragment thereof;
(12) an antibody in which CDR1, CDR2 and CDR3 of a heavy chain comprise amino acid sequences set forth as SEQ ID NOS: 68, 69 and 70, respectively, and CDR1, CDR2 and CDR3 of a light chain comprise amino acid sequences set forth as SEQ ID NOS: 71, 72 and 73, respectively, or a fragment thereof;
(13) an antibody in which CDR1, CDR2 and CDR3 of a heavy chain comprise amino acid sequences set forth as SEQ ID NOS: 74, 75 and 76, respectively, and CDR1, CDR2 and CDR3 of a light chain comprise amino acid sequences set forth as SEQ ID NOS: 77, 78 and 79, respectively, or a fragment thereof;
(14) an antibody in which CDR1, CDR2 and CDR3 of a heavy chain comprise amino acid sequences set forth as SEQ ID NOS: 80, 81 and 82, respectively, and CDR1, CDR2 and CDR3 of a light chain comprise amino acid sequences set forth as SEQ ID NOS: 83, 84 and 85, respectively, or a fragment thereof;
(15) an antibody in which CDR1, CDR2 and CDR3 of a heavy chain comprise amino acid sequences set forth as SEQ ID NOS: 86, 87 and 88, respectively, and CDR1, CDR2 and CDR3 of a light chain comprise amino acid sequences set forth as SEQ ID NOS: 89, 90 and 91, respectively, or a fragment thereof;
(16) an antibody in which CDR1, CDR2 and CDR3 of a heavy chain comprise amino acid sequences set forth as SEQ ID NOS: 92, 93 and 94, respectively, and CDR1, CDR2 and CDR3 of a light chain comprise amino acid sequences set forth as SEQ ID NOS: 95, 96 and 97, respectively, or a fragment thereof;
(17) an antibody in which CDR1, CDR2 and CDR3 of a heavy chain comprise amino acid sequences set forth as SEQ ID NOS: 98, 99 and 100, respectively, and CDR1, CDR2 and CDR3 of a light chain comprise amino acid sequences set forth as SEQ ID NOS: 101, 102 and 103, respectively, or a fragment thereof;
(18) an antibody in which CDR1, CDR2 and CDR3 of a heavy chain comprise amino acid sequences set forth as SEQ ID NOS: 104, 105 and 106, respectively, and CDR1, CDR2 and CDR3 of a light chain comprise amino acid sequences set forth as SEQ ID NOS: 107, 108 and 109, respectively, or a fragment thereof;
(19) an antibody in which CDR1, CDR2 and CDR3 of a heavy chain comprise amino acid sequences set forth as SEQ ID NOS: 110, 111 and 112, respectively, and CDR1, CDR2 and CDR3 of a light chain comprise amino acid sequences set forth as SEQ ID NOS: 113, 114 and 115, respectively, or a fragment thereof; and
(20) an antibody in which CDR1, CDR2 and CDR3 of a heavy chain comprise amino acid sequences set forth as SEQ ID NOS: 116, 117 and 118, respectively, and CDR1, CDR2 and CDR3 of a light chain comprise amino acid sequences set forth as SEQ ID NOS: 119, 120 and 121, respectively, or a fragment thereof.

6. The method for detecting SARS-CoV-2 according to any one of claims 1 to 4, wherein the test sample is a biological sample.

7. An immunochromatographic test strip for detecting SARS-CoV-2, comprising a first antibody or fragment thereof capable of reacting with a nucleocapsid protein of SARS-CoV-2, a second antibody or fragment thereof that reacts with the nucleocapsid protein, and a membrane carrier, wherein the first antibody or fragment thereof is previously fixed at a predetermined position on the membrane carrier to form a capture portion, and the second antibody or fragment thereof is labeled with a labeling substance and provided at a distance from the capture portion so as to be chromatographically developable in the membrane carrier.

8. The immunochromatographic test strip according to claim 7, wherein at least one of the first antibody or fragment thereof and the second antibody or fragment thereof is a monoclonal antibody or fragment thereof.

9. The immunochromatographic test strip according to claim 7 or 8, wherein the monoclonal antibody or fragment thereof is selected from the antibodies or fragments thereof described in (1) to (20) below:
(1) an antibody in which CDR1, CDR2 and CDR3 of a heavy chain comprise amino acid sequences set forth as SEQ ID NOS: 2, 3 and 4, respectively, and CDR1, CDR2 and CDR3 of a light chain comprise amino acid sequences set forth as SEQ ID NOS: 5, 6 and 7, respectively, or a fragment thereof;
(2) an antibody in which CDR1, CDR2 and CDR3 of a heavy chain comprise amino acid sequences set forth as SEQ ID NOS: 8, 9 and 10, respectively, and CDR1, CDR2 and CDR3 of a light chain comprise amino acid sequences set forth as SEQ ID NOS: 11, 12 and 13, respectively, or a fragment thereof;
(3) an antibody in which CDR1, CDR2 and CDR3 of a heavy chain comprise amino acid sequences set forth as SEQ ID NOS: 14, 15 and 16, respectively, and CDR1, CDR2 and CDR3 of a light chain comprise amino acid sequences set forth as SEQ ID NOS: 17, 18 and 19, respectively, or a fragment thereof;
(4) an antibody in which CDR1, CDR2 and CDR3 of a heavy chain comprise amino acid sequences set forth as SEQ ID NOS: 20, 21 and 22, respectively, and CDR1, CDR2 and CDR3 of a light chain comprise amino acid sequences set forth as SEQ ID NOS: 23, 24 and 25, respectively, or a fragment thereof;
(5) an antibody in which CDR1, CDR2 and CDR3 of a heavy chain comprise amino acid sequences set forth as SEQ ID NOS: 26, 27 and 28, respectively, and CDR1, CDR2 and CDR3 of a light chain comprise amino acid sequences set forth as SEQ ID NOS: 29, 30 and 31, respectively, or a fragment thereof;
(6) an antibody in which CDR1, CDR2 and CDR3 of a heavy chain comprise amino acid sequences set forth as SEQ ID NOS: 32, 33 and 34, respectively, and CDR1, CDR2 and CDR3 of a light chain comprise amino acid sequences set forth as SEQ ID NOS: 35, 36 and 37, respectively, or a fragment thereof;
(7) an antibody in which CDR1, CDR2 and CDR3 of a heavy chain comprise amino acid sequences set forth as SEQ ID NOS: 38, 39 and 40, respectively, and CDR1, CDR2 and CDR3 of a light chain comprise amino acid sequences set forth as SEQ ID NOS: 41, 42 and 43, respectively, or a fragment thereof;
(8) an antibody in which CDR1, CDR2 and CDR3 of a heavy chain comprise amino acid sequences set forth as SEQ ID NOS: 44, 45 and 46, respectively, and CDR1, CDR2 and CDR3 of a light chain comprise amino acid sequences set forth as SEQ ID NOS: 47, 48 and 49, respectively, or a fragment thereof;
(9) an antibody in which CDR1, CDR2 and CDR3 of a heavy chain comprise amino acid sequences set forth as SEQ ID NOS: 50, 51 and 52, respectively, and CDR1, CDR2 and CDR3 of a light chain comprise amino acid sequences set forth as SEQ ID NOS: 53, 54 and 55, respectively, or a fragment thereof;
(10) an antibody in which CDR1, CDR2 and CDR3 of a heavy chain comprise amino acid sequences set forth as SEQ ID NOS: 56, 57 and 58, respectively, and CDR1, CDR2 and CDR3 of a light chain comprise amino acid sequences set forth as SEQ ID NOS: 59, 60 and 61, respectively, or a fragment thereof;
(11) an antibody in which CDR1, CDR2 and CDR3 of a heavy chain comprise amino acid sequences set forth as SEQ ID NOS: 62, 63 and 64, respectively, and CDR1, CDR2 and CDR3 of a light chain comprise amino acid sequences set forth as SEQ ID NOS: 65, 66 and 67, respectively, or a fragment thereof;
(12) an antibody in which CDR1, CDR2 and CDR3 of a heavy chain comprise amino acid sequences set forth as SEQ ID NOS: 68, 69 and 70, respectively, and CDR1, CDR2 and CDR3 of a light chain comprise amino acid sequences set forth as SEQ ID NOS: 71, 72 and 73, respectively, or a fragment thereof;
(13) an antibody in which CDR1, CDR2 and CDR3 of a heavy chain comprise amino acid sequences set forth as SEQ ID NOS: 74, 75 and 76, respectively, and CDR1, CDR2 and CDR3 of a light chain comprise amino acid sequences set forth as SEQ ID NOS: 77, 78 and 79, respectively, or a fragment thereof;
(14) an antibody in which CDR1, CDR2 and CDR3 of a heavy chain comprise amino acid sequences set forth as SEQ ID NOS: 80, 81 and 82, respectively, and CDR1, CDR2 and CDR3 of a light chain comprise amino acid sequences set forth as SEQ ID NOS: 83, 84 and 85, respectively, or a fragment thereof;
(15) an antibody in which CDR1, CDR2 and CDR3 of a heavy chain comprise amino acid sequences set forth as SEQ ID NOS: 86, 87 and 88, respectively, and CDR1, CDR2 and CDR3 of a light chain comprise amino acid sequences set forth as SEQ ID NOS: 89, 90 and 91, respectively, or a fragment thereof;
(16) an antibody in which CDR1, CDR2 and CDR3 of a heavy chain comprise amino acid sequences set forth as SEQ ID NOS: 92, 93 and 94, respectively, and CDR1, CDR2 and CDR3 of a light chain comprise amino acid sequences set forth as SEQ ID NOS: 95, 96 and 97, respectively, or a fragment thereof;
(17) an antibody in which CDR1, CDR2 and CDR3 of a heavy chain comprise amino acid sequences set forth as SEQ ID NOS: 98, 99 and 100, respectively, and CDR1, CDR2 and CDR3 of a light chain comprise amino acid sequences set forth as SEQ ID NOS: 101, 102 and 103, respectively, or a fragment thereof;
(18) an antibody in which CDR1, CDR2 and CDR3 of a heavy chain comprise amino acid sequences set forth as SEQ ID NOS: 104, 105 and 106, respectively, and CDR1, CDR2 and CDR3 of a light chain comprise amino acid sequences set forth as SEQ ID NOS: 107, 108 and 109, respectively, or a fragment thereof;
(19) an antibody in which CDR1, CDR2 and CDR3 of a heavy chain comprise amino acid sequences set forth as SEQ ID NOS: 110, 111 and 112, respectively, and CDR1, CDR2 and CDR3 of a light chain comprise amino acid sequences set forth as SEQ ID NOS: 113, 114 and 115, respectively, or a fragment thereof; and
(20) an antibody in which CDR1, CDR2 and CDR3 of a heavy chain comprise amino acid sequences set forth as SEQ ID NOS: 116, 117 and 118, respectively, and CDR1, CDR2 and CDR3 of a light chain comprise amino acid sequences set forth as SEQ ID NOS: 119, 120 and 121, respectively, or a fragment thereof.

10. A SARS-CoV-2 detection kit comprising at least the immunochromatographic test strip for detecting SARS-CoV-2 according to claim 7, and a developing solvent for developing an analyte.

11. An antibody or fragment thereof for detecting a presence of SARS-CoV-2 contained in a test sample, comprising an antibody or fragment thereof that binds to a nucleocapsid protein of the SARS-CoV-2.

12. The antibody or fragment thereof according to claim 11, wherein the antibody is a monoclonal antibody.

13. The antibody or fragment thereof according to claim 12, wherein CDR1, CDR2 and CDR3 of a heavy chain comprise amino acid sequences set forth as SEQ ID NOS: 2, 3 and 4, respectively, and CDR1, CDR2 and CDR3 of a light chain comprise amino acid sequences set forth as SEQ ID NOS: 5, 6 and 7, respectively.

14. The antibody or fragment thereof according to claim 12, wherein CDR1, CDR2 and CDR3 of a heavy chain comprise amino acid sequences set forth as SEQ ID NOS: 8, 9 and 10, respectively, and CDR1, CDR2 and CDR3 of a light chain comprise amino acid sequences set forth as SEQ ID NOS: 11, 12 and 13, respectively.

15. The antibody or fragment thereof according to claim 12, wherein CDR1, CDR2 and CDR3 of a heavy chain comprise amino acid sequences set forth as SEQ ID NOS: 14, 15 and 16, respectively, and CDR1, CDR2 and CDR3 of a light chain comprise amino acid sequences set forth as SEQ ID NOS: 17, 18 and 19, respectively.

16. The antibody or fragment thereof according to claim 12, wherein CDR1, CDR2 and CDR3 of a heavy chain comprise amino acid sequences set forth as SEQ ID NOS: 20, 21 and 22, respectively, and CDR1, CDR2 and CDR3 of a light chain comprise amino acid sequences set forth as SEQ ID NOS: 23, 24 and 25, respectively.

17. The antibody or fragment thereof according to claim 12, wherein CDR1, CDR2 and CDR3 of a heavy chain comprise amino acid sequences set forth as SEQ ID NOS: 26, 27 and 28, respectively, and CDR1, CDR2 and CDR3 of a light chain comprise amino acid sequences set forth as SEQ ID NOS: 29, 30 and 31, respectively.

18. The antibody or fragment thereof according to claim 12, wherein CDR1, CDR2 and CDR3 of a heavy chain comprise amino acid sequences set forth as SEQ ID NOS: 32, 33 and 34, respectively, and CDR1, CDR2 and CDR3 of a light chain comprise amino acid sequences set forth as SEQ ID NOS: 35, 36 and 37, respectively.

19. The antibody or fragment thereof according to claim 12, wherein CDR1, CDR2 and CDR3 of a heavy chain comprise amino acid sequences set forth as SEQ ID NOS: 38, 39 and 40, respectively, and CDR1, CDR2 and CDR3 of a light chain comprise amino acid sequences set forth as SEQ ID NOS: 41, 42 and 43, respectively.

20. The antibody or fragment thereof according to claim 12, wherein CDR1, CDR2 and CDR3 of a heavy chain comprise amino acid sequences set forth as SEQ ID NOS: 44, 45 and 46, respectively, and CDR1, CDR2 and CDR3 of a light chain comprise amino acid sequences set forth as SEQ ID NOS: 47, 48 and 49, respectively.

21. The antibody or fragment thereof according to claim 12, wherein CDR1, CDR2 and CDR3 of a heavy chain comprise amino acid sequences set forth as SEQ ID NOS: 50, 51 and 52, respectively, and CDR1, CDR2 and CDR3 of a light chain comprise amino acid sequences set forth as SEQ ID NOS: 53, 54 and 55, respectively.

22. The antibody or fragment thereof according to claim 12, wherein CDR1, CDR2 and CDR3 of a heavy chain comprise amino acid sequences set forth as SEQ ID NOS: 56, 57 and 58, respectively, and CDR1, CDR2 and CDR3 of a light chain comprise amino acid sequences set forth as SEQ ID NOS: 59, 60 and 61, respectively.

23. The antibody or fragment thereof according to claim 12, wherein CDR1, CDR2 and CDR3 of a heavy chain comprise amino acid sequences set forth as SEQ ID NOS: 62, 63 and 64, respectively, and CDR1, CDR2 and CDR3 of a light chain comprise amino acid sequences set forth as SEQ ID NOS: 65, 66 and 67, respectively.

24. The antibody or fragment thereof according to claim 12, wherein CDR1, CDR2 and CDR3 of a heavy chain comprise amino acid sequences set forth as SEQ ID NOS: 68, 69 and 70, respectively, and CDR1, CDR2 and CDR3 of a light chain comprise amino acid sequences set forth as SEQ ID NOS: 71, 72 and 73, respectively.

25. The antibody or fragment thereof according to claim 12, wherein CDR1, CDR2 and CDR3 of a heavy chain comprise amino acid sequences set forth as SEQ ID NOS: 74, 75 and 76, respectively, and CDR1, CDR2 and CDR3 of a light chain comprise amino acid sequences set forth as SEQ ID NOS: 77, 78 and 79, respectively.

26. The antibody or fragment thereof according to claim 12, wherein CDR1, CDR2 and CDR3 of a heavy chain comprise amino acid sequences set forth as SEQ ID NOS: 80, 81 and 82, respectively, and CDR1, CDR2 and CDR3 of a light chain comprise amino acid sequences set forth as SEQ ID NOS: 83, 84 and 85, respectively.

27. The antibody or fragment thereof according to claim 12, wherein CDR1, CDR2 and CDR3 of a heavy chain comprise amino acid sequences set forth as SEQ ID NOS: 86, 87 and 88, respectively, and CDR1, CDR2 and CDR3 of a light chain comprise amino acid sequences set forth as SEQ ID NOS: 89, 90 and 91, respectively.

28. The antibody or fragment thereof according to claim 12, wherein CDR1, CDR2 and CDR3 of a heavy chain comprise amino acid sequences set forth as SEQ ID NOS: 92, 93 and 94, respectively, and CDR1, CDR2 and CDR3 of a light chain comprise amino acid sequences set forth as SEQ ID NOS: 95, 96 and 97, respectively.

29. The antibody or fragment thereof according to claim 12, wherein CDR1, CDR2 and CDR3 of a heavy chain comprise amino acid sequences set forth as SEQ ID NOS: 98, 99 and 100, respectively, and CDR1, CDR2 and CDR3 of a light chain comprise amino acid sequences set forth as SEQ ID NOS: 101, 102 and 103, respectively.

30. The antibody or fragment thereof according to claim 12, wherein CDR1, CDR2 and CDR3 of a heavy chain comprise amino acid sequences set forth as SEQ ID NOS: 104, 105 and 106, respectively, and CDR1, CDR2 and CDR3 of a light chain comprise amino acid sequences set forth as SEQ ID NOS: 107, 108 and 109, respectively.

31. The antibody or fragment thereof according to claim 12, wherein CDR1, CDR2 and CDR3 of a heavy chain comprise amino acid sequences set forth as SEQ ID NOS: 110, 111 and 112, respectively, and CDR1, CDR2 and CDR3 of a light chain comprise amino acid sequences set forth as SEQ ID NOS: 113, 114 and 115, respectively.

32. The antibody or fragment thereof according to claim 12, wherein CDR1, CDR2 and CDR3 of a heavy chain comprise amino acid sequences set forth as SEQ ID NOS: 116, 117 and 118, respectively, and CDR1, CDR2 and CDR3 of a light chain comprise amino acid sequences set forth as SEQ ID NOS: 119, 120 and 121, respectively.
